# EUROPEAN PATENT APPLICATION

(11) **EP 4 574 033 A1**
(43) Date of publication of application: **25.06.2025**
(21) Application number: 23878905.1
(22) Date of filing: 21.09.2023
(51) Int. Cl.: A61B 5/107

(54) **BODY SHAPE MEASUREMENT METHOD AND ELECTRONIC DEVICE**

(30) Priority: 20.10.2022 CN 202211289414
(71) Applicant: Huawei Technologies Co., Ltd., Shenzhen, Guangdong 518129 (CN)
(72) Inventor: ZHAO, Jie, Shenzhen, Guangdong 518129 (CN); CHEN, Xiaohan, Shenzhen, Guangdong 518129 (CN); MA, Chunhui, Shenzhen, Guangdong 518129 (CN); HUANG, Lei, Shenzhen, Guangdong 518129 (CN); LIU, Hang, Shenzhen, Guangdong 518129 (CN); WEI, Peng, Shenzhen, Guangdong 518129 (CN)
(74) Representative: Pfenning, Meinig & Partner mbB
(86) International application number: PCT/CN2023/120424
(87) International publication number: WO 2024/082912

(57) **Abstract**

This application relates to the field of electronic device technologies, and provides a body shape measurement method and an electronic device, to improve accuracy of obtained body shape data, so that the obtained body shape data is more consistent with to an actual body shape of a user. The method is applied to the electronic device, and includes: obtaining a first body shape measurement parameter, where the first body shape measurement parameter includes user information and a user body image; determining a first target compensation amount based on first historical modification data, where the first historical modification data is an amount of modification by a user to historically measured body shape data; determining first body shape data based on the first body shape measurement parameter and the first target compensation amount; and outputting the first body shape data. Further, different fitness and health courses may be recommended to the user based on the measured body shape data.

## Description

This application claims priority to Chinese Patent Application No. 202211289414.1, filed with the China National Intellectual Property Administration on October 20, 2022 and entitled "BODY SHAPE MEASUREMENT METHOD AND ELECTRONIC DEVICE", which is incorporated herein by reference in its entirety.

### TECHNICAL FIELD

This application relates to the field of electronic device technologies, and in particular, to a body shape measurement method and an electronic device.

### BACKGROUND

As people's health awareness increases progressively, a series of problems such as hypertension and out-of-shape caused by obesity are increasingly concerned. To cope with the series of problems, exercise-based fat loss gradually becomes an indispensable part of people's daily life. A process and an effect of exercise-based fat loss may be presented by using body shape data. Therefore, how to obtain body shape data with high accuracy is a problem that needs to be resolved urgently.

### SUMMARY

This application provides a body shape measurement method and an electronic device, to improve accuracy of obtained body shape data, so that the obtained body shape data is more consistent with an actual body shape of a user.

To achieve the foregoing objective, the following technical solutions are used in this application.

According to a first aspect, this application provides a body shape measurement method, applied to an electronic device. The method includes: obtaining a first body shape measurement parameter, where the first body shape measurement parameter includes user information and a user body image; determining a target compensation amount based on first historical modification data, where the first historical modification data is an amount of modification by a user to historically measured body shape data; determining first body shape data based on the first body shape measurement parameter and the first target compensation amount; and outputting the first body shape data.

Based on the foregoing technical solution, the body shape data of the user is determined by using the user information, the user body image, and the amount of modification by the user to the historically measured body shape data. The user modifies the obtained body shape data. This means that the measured body shape data may have a specific error. In this way, the body shape data is determined based on an amount of historical modification by the user for body shape data. In other words, the amount of modification by the user to the body shape data is compensated to the obtained body shape data. This can reduce the error of the obtained body shape data, improve accuracy of the body shape data, and reduce a quantity of modifications made by the user to the body shape data, thereby bringing convenience to the user.

In a possible design, after the outputting the first body shape data, the method further includes: receiving a first user operation. The first user operation is used to modify the first body shape data, and a range in which the first body shape data is modifiable is a first modification range. Based on this design, the user may modify, in the first modification range, body shape data output this time by the electronic device. In other words, a modification range for the user is limited. This can prevent reduction of accuracy of the obtained body shape data because the user randomly or incorrectly modifies the output body shape data.

In a possible design, after the outputting the first body shape data, the method further includes: obtaining a second body shape measurement parameter, where the second body shape measurement parameter includes user information and a user body image; determining a second target compensation amount based on second historical modification data, where the second historical modification data is an amount of modification by the user to historically measured body shape data; determining second body shape data based on the second body shape measurement parameter and the second target compensation amount; and outputting the second body shape data.

In a possible design, after the outputting the second body shape data, the method further includes: receiving a second user operation. The second user operation is used to modify the second body shape data. A range in which the second body shape data is modifiable is a second modification range, and the second modification range is different from first modification range.

Based on this design, the user may modify, in the second modification range, body shape data output this time by the electronic device. In other words, a modification range for the user is limited. This can prevent reduction of accuracy of the obtained body shape data because the user randomly or incorrectly modifies the output body shape data. In addition, the first modification range is different from the second modification range. In other words, during different body shape measurement operations, modification ranges for the user may be different. Because the amount of manual modification by the user is compensated to the obtained body shape data, the amount of manual modification by the user gradually decreases. In this way, during different body shape measurement operations, an appropriate modification range is configured. For example, the second modification range is smaller than the first modification range. In this way, accuracy of the obtained body shape data can be further improved.

In a possible design, the first modification range is determined based on an error sample, and the error sample is an error between body shape data measured by the electronic device and actual body shape data of the user.

Based on this design, at an early phase of measurement, due to a measurement error, an amount of modification by the user to the body shape data may be large. The error sample is an error between the body shape data measured by the electronic device and the actual body shape data of the user. Therefore, the modification range for the user is determined based on the error sample, to ensure that the modification range for the user is within an error range. This ensures that the user can manually compensate for the measurement error of the electronic device, and prevents reduction of accuracy of the obtained body shape data because the user randomly or incorrectly modifies the body shape data output by a mobile phone.

In a possible design, the error sample satisfies a normal distribution in which an expectation is u and a variance is σ2, and the first modification range is determined based on u and σ2. Based on this design, the error sample may be in the normal distribution, and the modification range for the user is determined based on the expectation and the variance of the normal distribution, so that an error with a high probability can be used as the modification range for the user. The error with a high probability is a most possible error between the body shape data measured by the electronic device and the actual body shape data. In this way, the user modifies the body shape data in the modification range. In this way, accuracy of the obtained body shape data can be further improved, so that the obtained body shape data is more consistent with the actual body shape of the user.

In a possible design, the second modification range is determined based on a plurality of types of information in third body shape data, the second body shape data, a first body weight change amount, and a predicted error. The third body shape data is body shape data measured last time before the second body shape data is measured by the electronic device. The first body weight change amount is a change amount of a body weight corresponding to the second body shape data relative to a body weight corresponding to the third body shape data. The predicted error is an error between estimated second body shape data and the actual body shape data.

Based on this design, the modification range for the user is determined based on the plurality of types of information in the body shape data measured this time, the body shape data measured last time, the body weight change amounts respectively corresponding to the body shape data measured this time and the body shape data measured last time, the predicted error, and the like. A change trend of the body shape data can be kept consistent with a change trend of the body weight as much as possible. For example, if the body weight increases, the body shape data also increases. In this way, accuracy of the obtained body shape data is further improved, so that the obtained body shape data is more consistent with a change of the actual body shape.

In a possible design, the second modification range is determined based on a plurality of types of information in an estimated body shape change amount, the predicted error, and the second body shape data. The estimated body shape change amount is a change amount of the estimated second body shape data relative to the third body shape data. The estimated body shape change amount and the first body weight change amount meet a first preset condition. Based on this design, the modification range for the user is determined based on the estimated body shape change amount, the predicted error, and the second body shape data. The estimated body shape change amount and the first body weight change amount meet the preset condition. In this way, a change trend of the body shape data can be kept consistent with a change trend of the body weight as much as possible. For example, if the body weight increases, the body shape data also increases. In this way, accuracy of the obtained body shape data is further improved, so that the obtained body shape data is more consistent with a change of the actual body shape.

In a possible design, the first preset condition includes positive correlation. Based on this design, the change of the body shape data can be consistent with the change trend of the body weight. This can further improve accuracy of the obtained body shape data, so that the obtained body shape data is more consistent with the change of the actual body shape.

In a possible design, the second modification range is determined based on a preset modification range. Based on this design, the second modification range is directly determined based on the preset modification range. This prevents reduction of accuracy of the obtained body shape data because the user randomly or incorrectly modifies the body shape data output by the mobile phone. In addition, this simplifies an operation of determining the modification range.

In a possible design, the determining first body shape data based on the first body shape measurement parameter and first target compensation amount includes: determining the first body shape data based on the first body shape measurement parameter, the first target compensation amount, and fourth body shape data, where the fourth body shape data includes the historically measured body shape data. Based on this design, final current body shape data is determined with reference to the historically obtained body shape data and the currently obtained body shape data, so that an accidental error of the body shape data determined this time can be reduced. In this way, when the body shape data is measured a plurality of times in a long term, fluctuation of the body shape data can be smaller, and the body shape data is more consistent with an actual situation.

In a possible design, the determining the first body shape data based on the first body shape measurement parameter, the first target compensation amount, and fourth body shape data includes: determining the first body shape data based on the first body shape measurement parameter, the first target compensation amount, the fourth body shape data, a second body weight change amount, and fifth body shape data. The second body weight change amount is a change amount of a body weight corresponding to the first body shape data relative to a body weight corresponding to the fifth body shape data, and the fifth body shape data is body shape data measured last time before the first body shape data is measured. Based on this design, the body shape data is determined with reference to the body weight change amount, so that a change trend of the body weight can be consistent with a change trend of the body shape data as much as possible. For example, both increase or decrease. In this way, the obtained body shape data is more accurate and more consistent with a real situation.

In a possible design, the user information includes one or more of a gender, a height, a body weight, an age, a shoe type, and a clothing type.

In a possible design, the method further includes: obtaining a reference factor; and when the reference factor meets a first condition, reminding the user to perform body shape measurement. Based on this design, when the reference factor meets the first condition, the user is reminded to actively perform body shape measurement, so that the user can perform a body shape measurement operation based on the reminder, to obtain body shape data of the user. In this way, the body shape data can be tracked as a long-term indicator, to reflect a body shape change of the user. In addition, compared with that in a case in which the body shape data of the user is not measured for a long time, in a case in which the user is reminded in a timely manner to perform body shape measurement, a body shape measurement frequency can be increased. In this way, precision of the target compensation amount, a range available for editing by the user, and the like can be improved, thereby improving precision of the obtained body shape data.

In a possible design, the reference factor includes one or more of the following: an amount of exercise, a third body weight change amount, and a period in which no body shape measurement is performed. The third body weight change amount is a change amount of a current body weight obtained by the electronic device relative to a body weight obtained last time. Because the amount of exercise, the body weight change amount, the period in which no body shape measurement is performed, and the like are closely related to the body shape data of the user, when these reference factors meet the first condition, it indicates that there is a high probability that the body shape data of the user changes. Therefore, reminding the user based on these reference factors can improve a reminding effect. This reduces a probability of poor user experience caused by frequent reminders for the user when the body shape data of the user does not change.

According to a second aspect, this application provides an electronic device. The electronic device has a function of implementing the method according to any one of the first aspect and the designs of the first aspect. The function may be implemented by hardware, or may be implemented by hardware executing corresponding software. The hardware or the software includes one or more modules corresponding to the function.

According to a third aspect, this application provides an electronic device, including a processor, a memory, and a display. The memory and the display are coupled to the processor. The memory is configured to store computer program code. The computer program code includes computer instructions. When the processor reads the computer instructions from the memory, the electronic device is enabled to perform the following operations: obtaining a first body shape measurement parameter, where the first body shape measurement parameter includes user information and a user body image; determining a target compensation amount based on first historical modification data, where the first historical modification data is an amount of modification by a user to historically measured body shape data; determining first body shape data based on the first body shape measurement parameter and the first target compensation amount; and outputting the first body shape data.

Optionally, the memory may be coupled to the processor, or may be independent of the processor.

In a possible design, when the processor reads the computer instructions from the memory, the electronic device is further enabled to perform the following operation: receiving a first user operation. The first user operation is used to modify the first body shape data, and a range in which the first body shape data is modifiable is a first modification range.

In a possible design, when the processor reads the computer instructions from the memory, the electronic device is further enabled to perform the following operations: obtaining a second body shape measurement parameter, where the second body shape measurement parameter includes user information and a user body image; determining a second target compensation amount based on second historical modification data, where the second historical modification data is an amount of modification by the user to historically measured body shape data; determining second body shape data based on the second body shape measurement parameter and the second target compensation amount; and outputting the second body shape data.

In a possible design, when the processor reads the computer instructions from the memory, the electronic device is further enabled to perform the following operation: receiving a second user operation. The second user operation is used to modify the second body shape data. A range in which the second body shape data is modifiable is a second modification range, and the second modification range is different from first modification range.

In a possible design, the first modification range is determined based on an error sample, and the error sample is an error between body shape data measured by the electronic device and actual body shape data of the user.

In a possible design, the error sample satisfies a normal distribution in which an expectation is u and a variance is σ2, and the first modification range is determined based on u and σ2.

In a possible design, the second modification range is determined based on a plurality of types of information in third body shape data, the second body shape data, a first body weight change amount, and a predicted error. The third body shape data is body shape data measured last time before the second body shape data is measured by the electronic device. The first body weight change amount is a change amount of a body weight corresponding to the second body shape data relative to a body weight corresponding to the third body shape data. The predicted error is an error between estimated second body shape data and the actual body shape data.

In a possible design, the second modification range is determined based on a plurality of types of information in an estimated body shape change amount, the predicted error, and the second body shape data. The estimated body shape change amount is a change amount of the estimated second body shape data relative to the third body shape data. The estimated body shape change amount and the first body weight change amount meet a first preset condition.

In a possible design, the first preset condition includes positive correlation.

In a possible design, the second modification range is determined based on a preset modification range.

In a possible design, the determining first body shape data based on the first body shape measurement parameter and first target compensation amount includes: determining the first body shape data based on the first body shape measurement parameter, the first target compensation amount, and fourth body shape data, where the fourth body shape data includes the historically measured body shape data.

In a possible design, the determining the first body shape data based on the first body shape measurement parameter, the first target compensation amount, and fourth body shape data includes: determining the first body shape data based on the first body shape measurement parameter, the first target compensation amount, the fourth body shape data, a second body weight change amount, and fifth body shape data. The second body weight change amount is a change amount of a body weight corresponding to the first body shape data relative to a body weight corresponding to the fifth body shape data, and the fifth body shape data is body shape data measured last time before the first body shape data is measured.

In a possible design, the user information includes one or more of a gender, a height, a body weight, an age, a shoe type, and a clothing type.

In a possible design, when the processor reads the computer instructions from the memory, the electronic device is further enabled to perform the following operations: obtaining a reference factor; and when the reference factor meets a first condition, reminding the user to perform body shape measurement.

In a possible design, the reference factor includes one or more of the following: an amount of exercise, a third body weight change amount, and a period in which no body shape measurement is performed. The third body weight change amount is a change amount of a current body weight obtained by the electronic device relative to a body weight obtained last time.

In a possible design, the electronic device further includes a communication interface, and the communication interface may be used by the electronic device to communicate with another apparatus. For example, the communication interface may be a transceiver, an input/output interface, an interface circuit, an output circuit, an input circuit, a pin, a related circuit, or the like.

According to a fourth aspect, this application provides a computer-readable storage medium. The computer-readable storage medium includes a computer program or instructions. When the computer program or the instructions are run on an electronic device, the electronic device is enabled to perform the method according to any one of the first aspect and the designs of the first aspect.

According to a fifth aspect, this application provides a computer program product. When the computer program product runs on a computer, the computer is enabled to perform the method according to any one of the first aspect and the designs of the first aspect.

According to a sixth aspect, this application provides a chip system, including at least one processor and at least one interface circuit. The at least one interface circuit is configured to: perform a transceiver function, and send instructions to the at least one processor, and when the at least one processor executes the instructions, the at least one processor performs the method according to any one of the first aspect and the designs of the first aspect.

It should be noted that, for technical effects achieved by any one of the designs of the second aspect to the sixth aspect, refer to the technical effects achieved by a corresponding design of the first aspect. Details are not described herein again.

### BRIEF DESCRIPTION OF DRAWINGS

FIG. 1 is a diagram of a structure of an electronic device according to an embodiment of this application;
FIG. 2 is a diagram of a structure of another electronic device according to an embodiment of this application;
FIG. 3(1) to FIG. 8 are interface diagrams 1 to 6 according to an embodiment of this application;
FIG. 9 is a schematic flowchart of a body shape measurement method according to an embodiment of this application;
FIG. 10 is a diagram of error distribution according to an embodiment of this application;
FIG. 11(1) to FIG. 11(4) is an interface diagram 7 according to an embodiment of this application;
FIG. 12 is a schematic flowchart of a method for determining a range available for editing according to an embodiment of this application;
FIG. 13 is an interface diagram 8 according to an embodiment of this application;
FIG. 14 is a schematic flowchart of a method for reminding a user to perform body shape measurement according to an embodiment of this application;
FIG. 15 is a schematic flowchart of another method for reminding a user to perform body shape measurement according to an embodiment of this application;
FIG. 16 is a bar chart of body shape data according to an embodiment of this application;
FIG. 17 is a diagram of a structure of still another electronic device according to an embodiment of this application; and
FIG. 18 is a diagram of a structure of a chip system according to an embodiment of this application.

### DESCRIPTION OF EMBODIMENTS

The following describes technical solutions in embodiments in this application in detail with reference to the accompanying drawings.

The terms "including", "having", and any other variant thereof mentioned in descriptions of this application are intended to cover a non-exclusive inclusion. For example, a process, a method, a system, a product, or a device that includes a series of steps or units is not limited to the listed steps or units, but optionally further includes other unlisted steps or units, or optionally further includes another inherent step or unit of the process, the method, the product, or the device.

It should be noted that, in embodiments of this application, the words "example" or "for example" indicate giving an example, an illustration, or a description. Any embodiment or design scheme described as an "example" or "for example" in embodiments of this application should not be explained as being more preferred or having more advantages than another embodiment or design scheme. Exactly, use of the words such as "example" or "for example" is intended to present a related concept in a specific manner.

In the descriptions of this application, unless otherwise specified, "a plurality of" means two or more than two. The term "and/or" in this specification describes only an association relationship between associated objects and indicates that there may be three relationships. For example, A and/or B may indicate the following three cases: Only A exists, both A and B exist, and only B exists.

An embodiment of this application provides a body shape measurement method, to improve accuracy of obtained body shape data. The body shape measurement method provided in this embodiment of this application may be applied to the electronic device 100, or applied to a system including an electronic device 100.

For example, the electronic device 100 may be various electronic devices having a body measurement function, such as a mobile phone, a tablet computer, a desktop computer, a laptop computer, a handheld computer, a notebook computer, an ultra-mobile personal computer (ultra-mobile personal computer, UMPC), a netbook, a personal digital assistant (personal digital assistant, PDA), an artificial intelligence (artificial intelligence, AI) device, a wearable device, a vehicle-mounted device, a smart home device, and/or a smart city device. An operating system installed on the electronic device 100 includes but is not limited to iOS^{®}, Android^{®}, Harmony^{®}, Windows^{®}, Linux^{®}, or another operating system. In some embodiments, the electronic device 100 may be a fixed device, or may be a portable device. A specific type of the electronic device 100 and the installed operating system are not limited in this application.

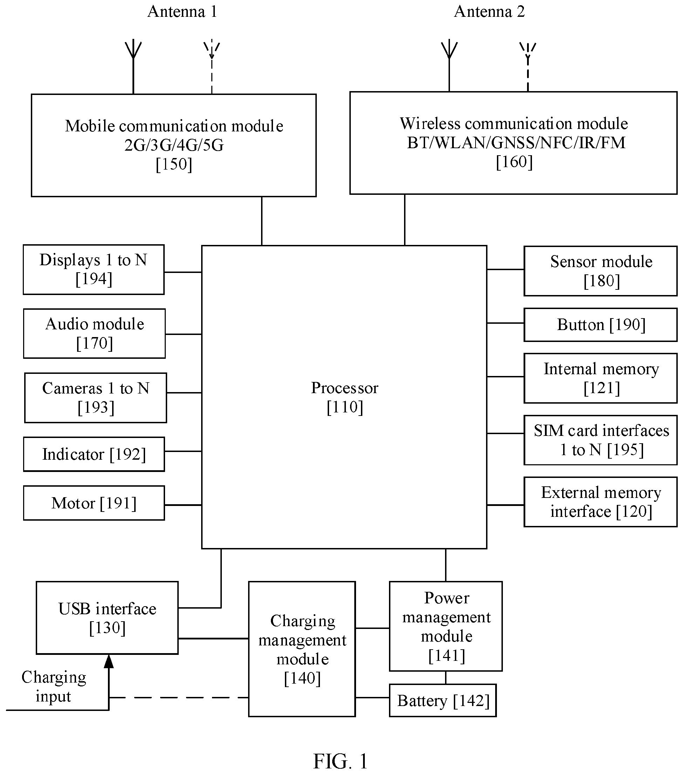

For example, the electronic device 100 is a mobile phone. FIG. 1 is a diagram of a structure of a mobile phone according to an embodiment of this application.

As shown in FIG. 1, the mobile phone may include a processor 110, an external memory interface 120, an internal memory 121, a universal serial bus (universal serial bus, USB) interface 130, a charging management module 140, a power management module 141, a battery 142, an antenna 1, an antenna 2, a mobile communication module 150, a wireless communication module 160, an audio module 170, a sensor module 180, a button 190, a motor 191, an indicator 192, a camera 193, a display 194, a subscriber identity module (subscriber identity module, SIM) card interface 195, and the like.

The processor 110 may include one or more processing units. For example, the processor 110 may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, a neural-network processing unit (neural-network processing unit, NPU), and/or the like. Different processing units may be independent components, or may be integrated into one or more processors.

The controller may generate an operation control signal based on an instruction operation code and a time sequence signal, to complete control of instruction fetching and instruction execution.

A memory may be further disposed in the processor 110, and is configured to store instructions and data. In some embodiments, the memory in the processor 110 is a cache memory. The memory may store instructions or data just used or cyclically used by the processor 110. If the processor 110 needs to use the instructions or the data again, the processor may directly invoke the instructions or the data from the memory. This avoids repeated access, and reduces waiting time of the processor 110, thereby improving system efficiency.

In some embodiments, the processor 110 may include one or more interfaces.

The charging management module 140 is configured to receive a charging input from a charger. The charger may be a wireless charger or a wired charger.

The power management module 141 is configured to connect to the battery 142, the charging management module 140, and the processor 110. The power management module 141 receives an input from the battery 142 and/or the charging management module 140, and supplies power to the processor 110, the internal memory 121, the display 194, the camera 193, the wireless communication module 160, and the like.

A wireless communication function of the mobile phone may be implemented by using the antenna 1, the antenna 2, the mobile communication module 150, the wireless communication module 160, the modem processor, the baseband processor, and the like.

The antenna 1 and the antenna 2 are configured to transmit and receive electromagnetic wave signals. Each antenna in the mobile phone may be configured to cover one or more communication frequency bands. Different antennas may be further multiplexed to improve antenna utilization. For example, the antenna 1 may be multiplexed as a diversity antenna of a wireless local area network. In some other embodiments, the antenna may be used in combination with a tuning switch.

The mobile communication module 150 may provide a wireless communication solution including 2G/3G/4G/5G, or the like applied to the mobile phone. The mobile communication module 150 may include at least one filter, a switch, a power amplifier, a low noise amplifier (low noise amplifier, LNA), and the like. In some embodiments, at least some functional modules in the mobile communication module 150 may be disposed in the processor 110. In some embodiments, at least some functional modules in the mobile communication module 150 and at least some modules in the processor 110 may be disposed in a same component.

The wireless communication module 160 may provide a wireless communication solution that is applied to the mobile phone and that includes a wireless local area network (wireless local area network, WLAN) (for example, a wireless fidelity (wireless fidelity, Wi-Fi) network), Bluetooth (bluetooth, BT), a global navigation satellite system (global navigation satellite system, GNSS), frequency modulation (frequency modulation, FM), a near-field communication (near-field communication, NFC) technology, an infrared (infrared, IR) technology, and the like. The wireless communication module 160 may be one or more components integrating at least one communication processing module.

In some embodiments, the antenna 1 and the mobile communication module 150 in the mobile phone are coupled, and the antenna 2 and the wireless communication module 160 in the mobile phone are coupled, so that the mobile phone can communicate with a network and another device by using a wireless communication technology. The wireless communication technology may include a global system for mobile communications (global system for mobile communications, GSM), a general packet radio service (general packet radio service, GPRS), code division multiple access (code division multiple access, CDMA), wideband code division multiple access (wideband code division multiple access, WCDMA), time-division code division multiple access (time-division code division multiple access, TD-SCDMA), long term evolution (long term evolution, LTE), BT, a GNSS, a WLAN, NFC, FM, an IR technology, and/or the like.

The mobile phone implements a display function by using the GPU, the display 194, the application processor, and the like. The GPU is a microprocessor for image processing, and is connected to the display 194 and the application processor. The GPU is configured to: perform mathematical and geometric computation, and render an image. The processor 110 may include one or more GPUs, which execute program instructions to generate or change display information.

The display 194 is configured to display an image, a video, and the like. The display 194 includes a display panel. In some embodiments, the mobile phone may include one or N displays 194, where N is a positive integer greater than 1. In some embodiments of this application, the display 194 may be configured to display body shape data.

The mobile phone may implement a photographing function by using the ISP, a camera 193, the video codec, the GPU, the display 194, the application processor, and the like.

The camera 193 is configured to capture a static image or a video. In some embodiments, the mobile phone may include one or N cameras 193, where N is a positive integer greater than 1. In some embodiments of this application, the camera may be configured to capture a human body image, and the captured human body image may be used to determine the body shape data.

The external memory interface 120 may be configured to be connected to an external storage card, for example, a micro SD card, to extend a storage capability of the mobile phone. The external memory card communicates with the processor 110 through the external memory interface 120, to implement a data storage function. For example, files such as music and videos are stored in the external storage card.

The internal memory 121 may be configured to store computer-executable program code. The executable program code includes instructions. The internal memory 121 may include a program storage area and a data storage area. The program storage area may store an operating system, an application required by at least one function (for example, a voice playing function or an image playing function), and the like. The data storage area may store data (for example, audio data or a phone book) created during use of the mobile phone. In addition, the internal memory 121 may include a high-speed random-access memory, or may include a nonvolatile memory, for example, at least one magnetic disk storage device, a flash memory, or a universal flash storage (universal flash storage, UFS). The processor 110 runs the instructions stored in the internal memory 121 and/or instructions stored in a memory disposed in the processor, to perform various function applications and data processing that are of the mobile phone. In some embodiments of this application, the internal memory 121 may store a body shape measurement model, and the body shape measurement model may be used to determine body shape data. For detailed descriptions of the body shape measurement model, refer to the following descriptions.

The mobile phone may implement an audio function, for example, music playing and recording, by using the audio module 170, the application processor, or the like.

The audio module 170 is configured to convert digital audio information into an analog audio signal for output, and is also configured to convert an analog audio input into a digital audio signal. The audio module 170 may be further configured to encode and decode an audio signal. In some embodiments, the audio module 170 may be disposed in the processor 110, or some functional modules in the audio module 170 are disposed in the processor 110. In some embodiments of this application, the audio module 170 may be configured to output a reminder message, to remind the user to perform body shape measurement.

Optionally, the sensor module 180 may include one or more of a pressure sensor, a gyroscope sensor, a barometric pressure sensor, a magnetic sensor, an acceleration sensor, a distance sensor, an optical proximity sensor, a fingerprint sensor, a temperature sensor, a touch sensor, an ambient light sensor, a bone conduction sensor, and the like.

The button 190 includes a power button, a volume button, and the like. The button 190 may be a mechanical button, or may be a touch button. The mobile phone may receive a button input, and generate a button signal input related to user settings and function control of the mobile phone.

The motor 191 may generate a vibration prompt. The motor 191 may be configured to provide an incoming call vibration prompt and a touch vibration feedback.

The indicator 192 may be an indicator light, and may be configured to indicate a charging status and a power change, or may be configured to indicate a message, a missed call, a notification, and the like.

The SIM card interface 195 is configured to connect to a SIM card.

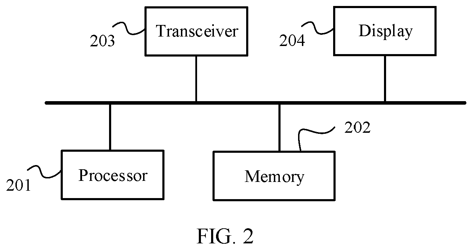

It may be understood that the structure of the electronic device 100 in this embodiment of this application is merely described above by using the mobile phone as an example, but does not constitute a limitation on a structure and a form of the electronic device 100. The structure and the form of the electronic device 100 are not limited in embodiments of this application. For example, FIG. 2 shows another example structure of the electronic device 100 according to an embodiment of this application. As shown in FIG. 2, the electronic device 100 includes a processor 201, a memory 202, a transceiver 203, and a display 204. For implementations of the processor 201, the memory 202, and the display 204, refer to the implementations of the processor, the memory, and the display of the mobile phone shown in FIG. 1. The transceiver 203 is configured for interaction between the electronic device 100 and another device. The transceiver 203 may be a component based on Wi-Fi, Bluetooth, or another communication protocol.

In some other embodiments of this application, the electronic device 100 may include more or fewer components than those shown in FIG. 1 and FIG. 2, or combine some components, or split some components, or have a different component arrangement. The components shown in the figure may be implemented by hardware, software, or a combination of software and hardware.

All technical solutions in the following embodiments may be implemented in devices having the structures shown in FIG. 1 and FIG. 2.

The following describes the technical solutions provided in embodiments of this application by using an example in which the electronic device 100 is a mobile phone with reference to the accompanying drawings.

In some embodiments, the mobile phone may determine body shape data of a user based on a user image (or referred to as a user body image).

For example, in this embodiment of this application, the body shape data of the user may include but is not limited to one or more of a body part length, a body part circumference, a body proportion, and the like. For example, the body part length may include but is not limited to a limb length (for example, a length of an arm, a leg, a thigh, a calf, an upper arm, a forearm, a hand, a palm, or a finger), a torso length, a neck length (or referred to as a length of the neck), and a head length. For example, the body part circumference may include but is not limited to a limb circumference (for example, a circumference of a thigh, a calf, an upper arm, a forearm, a wrist, or an ankle), a torso circumference (for example, a chest circumference, a waist circumference, a hip circumference, a shoulder circumference, a shoulder width, and a back width), a neck circumference (or referred to as a neck girth), and a head circumference. For example, the body proportion may include but is not limited to ratios between various body parts, such as a waist-hip ratio, a ratio of an upper body part to a lower body part, a head-neck ratio, a head-shoulder ratio, and a head-body ratio.

In some examples, the user image may be a full-body image of the user. For example, the full-body image of the user may include but is not limited to one or more of a front full-body image of the user, a back full-body image of the user, and a side full-body image of the user. It may be understood that, in this embodiment of this application, the front is a direction of the face of the user, and the back is a direction of the back of the user.

Optionally, in this example, the mobile phone may determine body shape data (for example, a length and a circumference) of one or more body parts of the user based on the full-body image of the user.

In some other examples, the user image may be a local-part image of the user. For example, the local-part image of the user may include but is not limited to an image of one or more to-be-measured parts, and an image of one or more parts other than the to-be-measured part. Optionally, the local-part image of the user may alternatively include one or more of a front local-part image of the user, a back local-part image of the user, a side local-part image of the user, and the like. For example, a part that the user wants to measure is the waist. In other words, the to-be-measured part is the waist. The local-part image of the user may include but is not limited to one or more of a waist image of the user, a hip image of the user, a face image of the user, and the like. For another example, the part that the user wants to measure is a thigh. In other words, the to-be-measured part is the thigh. The local-part image of the user may include but is not limited to one or more of a thigh image of the user, a calf image of the user, a hip image of the user, and the like.

Optionally, in this example, the mobile phone may alternatively determine body shape data of one or more body parts of the user based on the local-part image of the user. The mobile phone may determine body shape data of a to-be-measured part based on an image of the to-be-measured part, or may determine the body shape data of the to-be-measured part based on an image of a part other than the to-be-measured part.

Optionally, the mobile phone may capture the user image by using various sensors (for example, a camera), or may obtain the user image from another device. A manner of obtaining the user image by the mobile phone is not limited in this application.

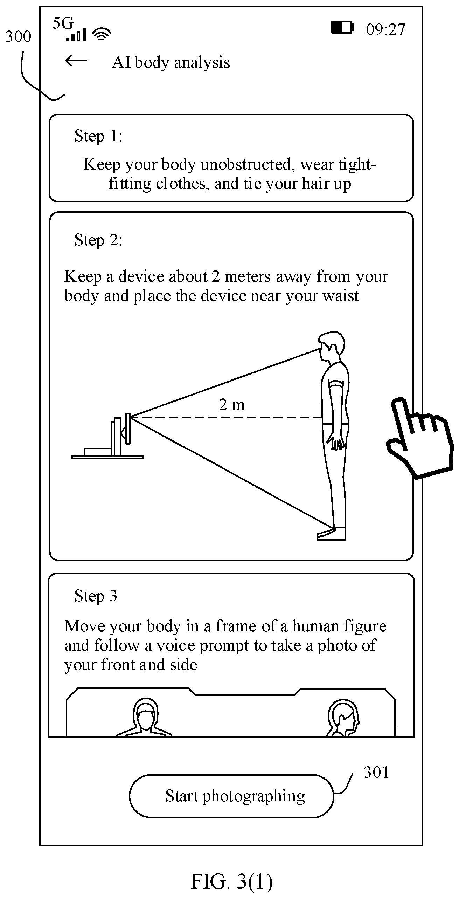

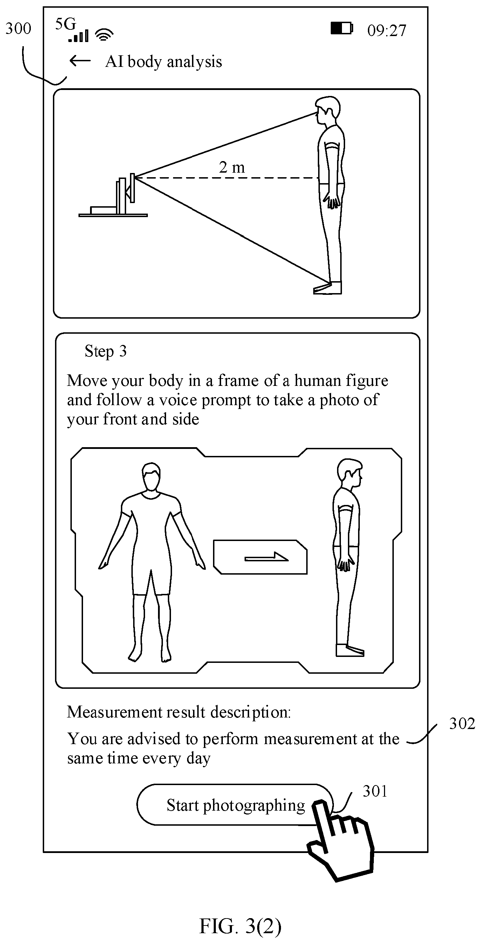

For example, the user image is a full-body image of the user. For example, FIG. 3(1) to FIG. 3(4) are diagrams of interfaces for capturing a user image by a mobile phone according to an embodiment of this application. As shown in FIG. 3(1) or FIG. 3(2), the mobile phone displays a body shape measurement interface 300. The body shape measurement interface 300 includes a photographing start button 301, and the photographing start button 301 may be used to start a camera of the mobile phone to capture a user image. Optionally, the body shape measurement interface 300 may further include an image capture step. The user may perform a correct operation based on the image capture step, so that the mobile phone can capture a correct user image.

The image capture step may be, for example, step 1 and step 2 shown in FIG. 3(1), or step 3 shown in FIG. 3(2). Optionally, because a display area of a display of the mobile phone is limited, an interface shown in FIG. 3(1) may not present all image capture steps. Therefore, the user may implement slidable display of the image capture steps through up-down sliding on the interface shown in FIG. 3(1). For example, if the mobile phone detects an operation of sliding up by the user on the interface shown in FIG. 3(1), in response to the operation, the mobile phone may present an interface shown in FIG. 3(2).

Optionally, as shown in FIG. 3(2), the body shape measurement interface 300 may further include a measurement result description 302 and the like, so that the user can select a correct measurement time point based on the measurement result description 302. In this way, measured body shape data of the user can truly reflect a body shape change and the like of the user.

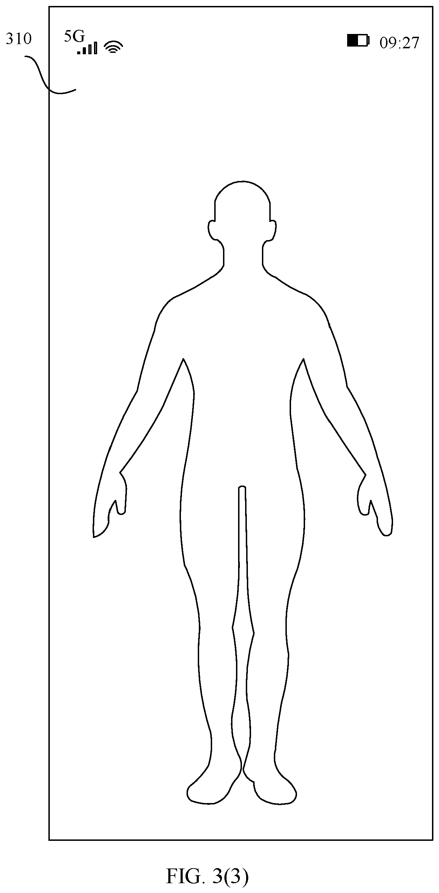

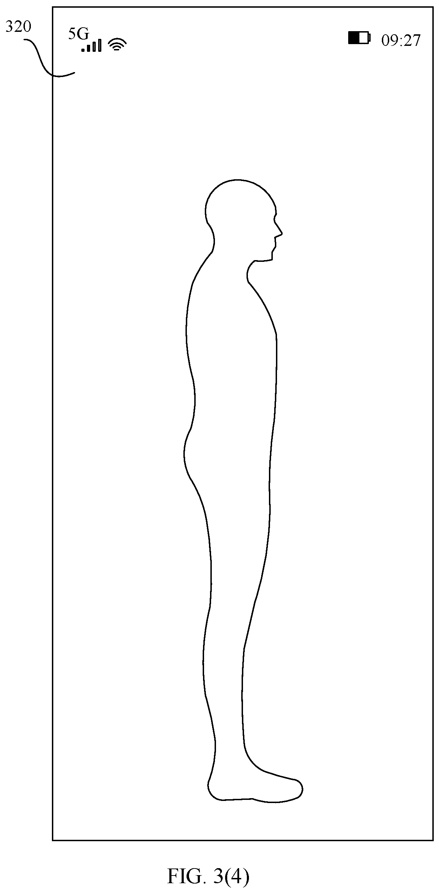

As shown in FIG. 3(2), the mobile phone detects, for example, a tap operation of the user on the photographing start button 301, and in response to the operation, the mobile phone starts the camera to capture a user image. For example, the mobile phone may present an image capture interface 310 shown in FIG. 3(3) for capturing a front full-body image of the user, and present an image capture interface 320 shown in FIG. 3(4) for capturing a side full-body image of the user. Optionally, the mobile phone may first present the image capture interface 310, or may first present the image capture interface 320. This is not limited in this application.

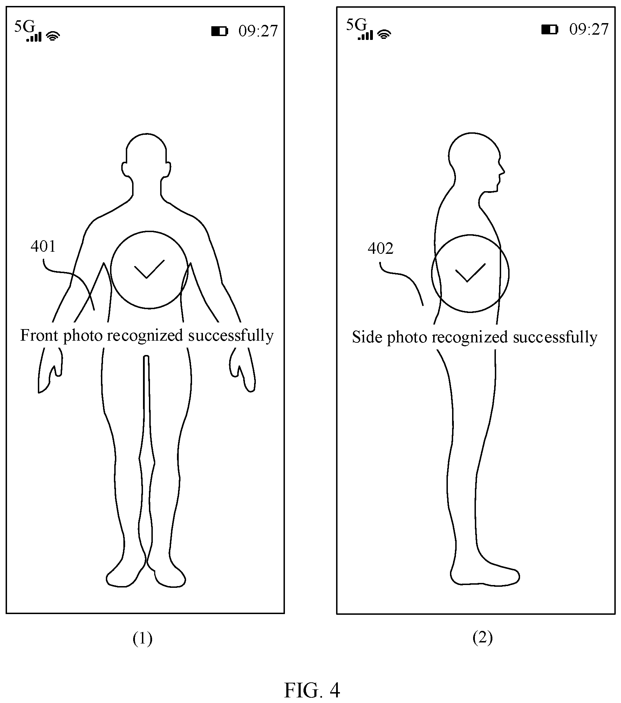

For example, the mobile phone first presents the image capture interface 310. Optionally, after the mobile phone successfully captures the front full-body image of the user, the mobile phone may further output a prompt message (for example, a prompt message broadcast by a speaker or a prompt message displayed on a display), to inform the user that the image is successfully captured. For example, the prompt message may be a prompt message 401 shown in (1) in FIG. 4. Then, the mobile phone may display, for example, an image capture interface 320 shown in FIG. 3(4) for capturing a side full-body image of the user. Certainly, after successfully capturing the side full-body image of the user, the mobile phone may also output a prompt message, to inform the user that the image is successfully captured. For example, the prompt message may be a prompt message 402 shown in (2) in FIG. 4.

Optionally, the mobile phone may input a user image to a first body shape measurement model, and output body shape data of the user by using the first body shape measurement model. The first body shape measurement model may be a model obtained through training by using a user image as an input and using user body shape data as an output.

It may be understood that the models in embodiments of this application may be various machine learning models and/or deep learning models. This is not limited in this application. Each model may be preset in the mobile phone, or may be preset in another device. This is not limited in this application either.

In some other embodiments, the mobile phone may further determine the body shape data of the user based on user information and a user image. For descriptions of the user image, refer to the descriptions in the foregoing embodiments.

For example, the user information may include but is not limited to one or more of a gender, a height, a body weight, an age, a shoe type, and a clothing type (or referred to as a clothes type). For example, the shoe type includes but is not limited to one or more of high heels, flat shoes, elevator shoes, and platform shoes. For descriptions of the clothing type, refer to the following descriptions.

In a possible example, the user information may be user information directly entered by the user into the mobile phone. For example, the mobile phone may receive, by using an installed application, the user information entered by the user. The installed application may be an application having a body shape measurement function, or may be an application having another function.

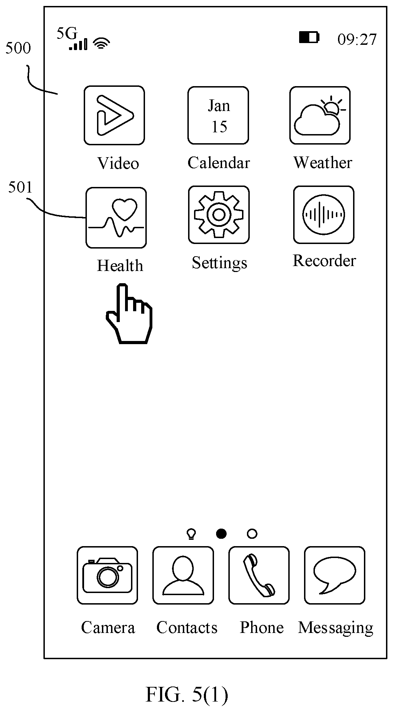

For example, the installed application is a Health application (hereinafter referred to as Health). For example, as shown in FIG. 5(1), the mobile phone displays a home screen 500 (or referred to as a desktop 500), and the home screen 500 includes icons of one or more applications, for example, an icon of a video application and an icon of a calendar application. Examples are not listed one by one herein. The icon of an application may be used to start a running interface of the corresponding application, to implement a function of the corresponding application. The icons of the plurality of applications include an icon 501 of Health for starting a running interface of Health.

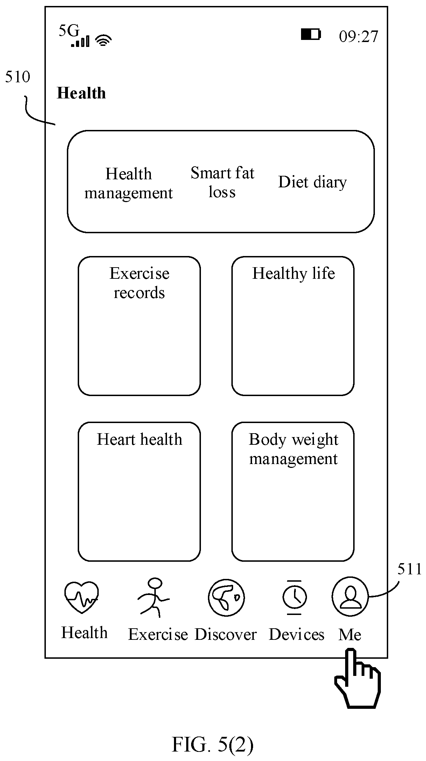

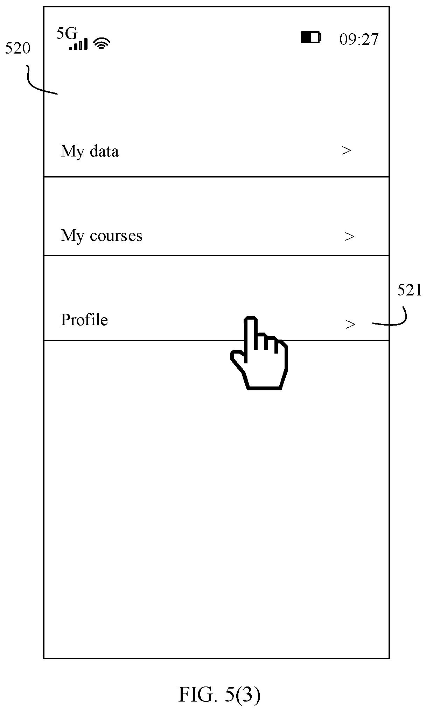

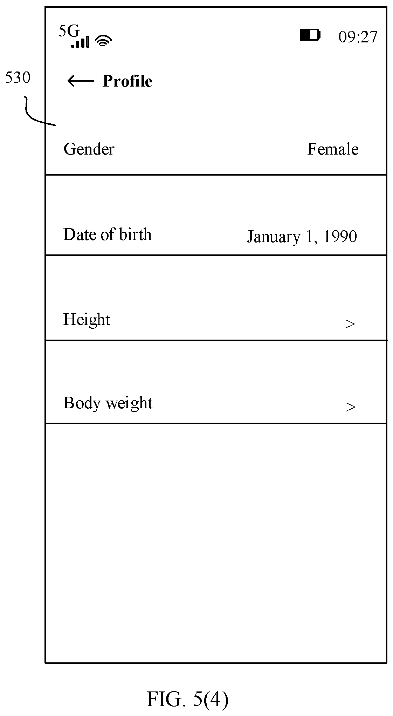

For example, the mobile phone detects a tap operation of the user on the icon 501 of Health. In response to the operation, as shown in FIG. 5(2), the mobile phone displays a running interface 510 of Health. The running interface 510 includes one or more functional modules such as health management, smart fat loss, exercise records, and heart health. Examples are not described one by one herein either. The mobile phone detects, for example, a tap operation of the user on a button 511. In response to the operation, as shown in FIG. 5(3), the mobile phone may display a personal interface 520. The personal interface 520 includes various options such as My data, My courses, and Profile, and different options may be used to view and/or set different information. The mobile phone detects, for example, a tap operation of the user on a profile option 521. In response to the operation, as shown in FIG. 5(4), the mobile phone may display a profile interface 530. The user may enter user information such as a gender, a height, and a body weight on the profile interface 530. Optionally, the user may further enter other information, for example, a date of birth. Correspondingly, the mobile phone may obtain the user information entered by the user on the profile interface 530.

In another possible example, the user information may alternatively be user information entered by the user into another device, and the mobile phone obtains the user information from the another device. For example, the another device may receive the user information in a manner shown in FIG. 5(1) to FIG. 5(4). Certainly, the another device may receive the user information in another manner. A manner of receiving the user information by the another device is not limited in this application.

In still another possible example, the user information may alternatively be user information measured by the mobile phone and/or another device. For example, the user information includes the body weight. When the user measures the body weight by using a weighing device like a body fat scale, a body weight of the user may be measured by the body fat scale. The mobile phone may be associated with the body fat scale to obtain the body weight of the user. For example, the user information includes the height. When the user uses a height measurement device like a height and body weight measurement instrument, a height of the user may be measured by the height and body weight measurement instrument. The mobile phone may be associated with the height and body weight measurement instrument to obtain the height of the user.

In still another possible example, the user information may alternatively be obtained through calculation by the mobile phone and/or another device based on other information. For example, the mobile phone and/or the another device may estimate the user information based on a captured user image and the like. For example, the user image may include a face image, and the mobile phone and/or the another device may determine the user information (for example, a gender and an age) by using a facial recognition algorithm. For another example, the user information is the height. The mobile phone and/or the another device may estimate a height of the user based on information such as a distance between the mobile phone and/or the another device and the user, a pixel proportion of the user in an image captured by the mobile phone and/or the another device, and a camera focal length. For example, the mobile phone and/or the another device may determine the distance between the mobile phone and/or the another device and the user by using a time of flight (time of flight, TOF) camera, a depth estimation algorithm, or the like. A manner of determining the distance between the mobile phone and/or the another device and the user is not limited in this application.

It may be understood that a manner of obtaining the user information by the mobile phone is not limited in embodiments of this application.

Optionally, the mobile phone may input the user image and the user information to a second body shape measurement model, and output body shape data of the user by using the second body shape measurement model. The second body shape measurement model is a model obtained through training by using user information and a user image as an input and using user body shape data as an output.

In some embodiments, because looseness of clothing varies, for example, clothing is excessively loose, body shape data of the user determined based on one or more of the user image, the user information, and the like may be inaccurate. Therefore, the mobile phone may further determine a looseness degree of clothing, and then determine the body shape data of the user based on the looseness degree of clothing and one or more of the user image and the user information.

For example, the mobile phone may determine the looseness degree of clothing based on the user image and a historical contour. The historical contour may be a user contour obtained when body shape data of the user is measured before the current measurement operation. Optionally, the user contour may be a full-body contour of the user, or may be a contour of a body part of the user. For example, if a difference between the user contour determined based on the user image and the historical contour is greater than or equal to a preset difference, it may be determined that the user wears loose clothes. If the difference between the user contour determined based on the user image and the historical contour is less than the preset difference, it may be determined that the user wears tight-fitting clothes.

Alternatively, the mobile phone may determine the looseness degree of clothing based on the clothing type. For example, the clothing type may include but is not limited to a sweatshirt, a sweater, a down jacket, a cotton-padded jacket, a camisole, a shirt, a skirt, palazzo pants, pencil pants, tights, and the like. For example, if the clothing type is the sweatshirt, the skirt, the palazzo pants, or the like, it may be determined that clothing is loose. If the clothing type is the camisole, the pencil pants, the tights, or the like, it may be determined that clothing is tight. Optionally, the clothing type may be determined by the mobile phone and/or another device by capturing a clothing image, or may be directly entered by the user.

Alternatively, the looseness degree of clothing may be directly entered by the user. A manner in which the mobile phone determining the looseness degree of clothing is not limited in this application.

In some examples, when determining that the user wears loose clothes, the mobile phone may further prompt the user to wear tight-fitting clothes, to obtain body shape data with high accuracy.

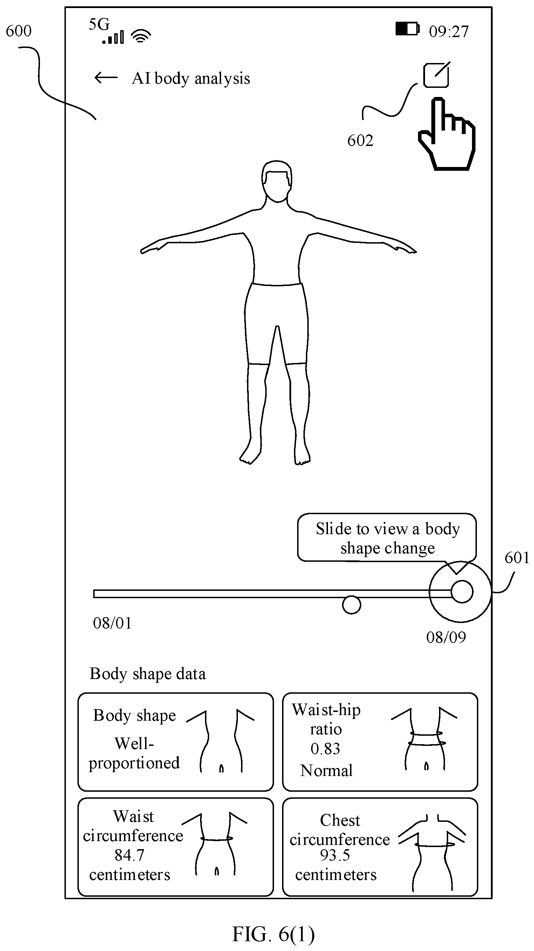

In some embodiments, after measuring the body shape data of the user, the mobile phone may output the body shape data of the user. For example, as shown in FIG. 6(1), the mobile phone may display a body shape data output interface 600, and the body shape data output interface 600 includes one or more pieces of body shape data, for example, a body shape (for example, various body shapes like a pear shape, an H shape, an apple shape, an hourglass shape, an O shape, an inverted triangle shape, and a well-proportioned shape), a body proportion (for example, a waist-hip ratio), and a body part circumference (for example, a chest circumference, a waist circumference, a hip circumference, an upper arm circumference, a thigh circumference, and a calf circumference). FIG. 6(1) shows only various kinds of body shape data, such as the body shape, the waist-hip ratio, the chest circumference, and the waist circumference. Optionally, the mobile phone may alternatively send the body shape data of the user to another device, and the another device outputs the body shape data of the user.

Optionally, the body shape data output interface 600 may further include a sliding bar 601. The user may view body shape data measured at different time points (for example, time points from August 1 to August 9) by performing operations, for example, sliding left or right on the sliding bar 601, to observe changes in the body shape.

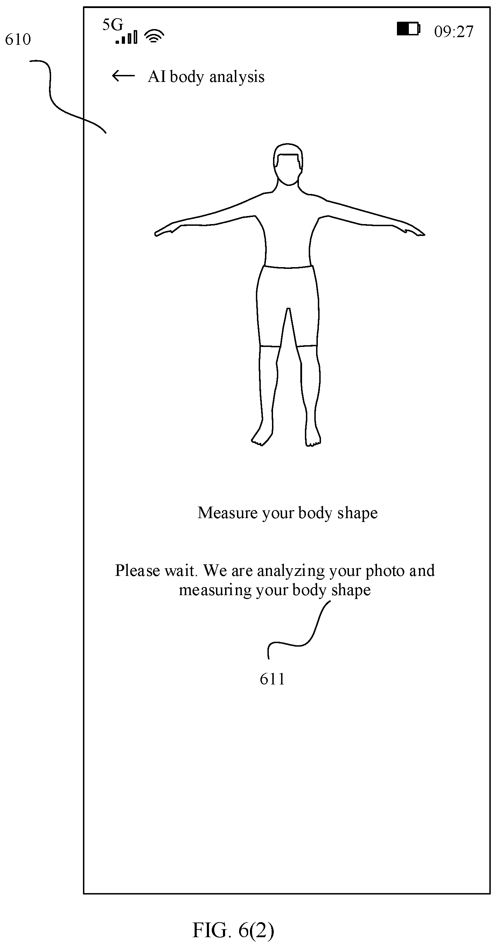

Optionally, before the mobile phone outputs the body shape data of the user, the mobile phone may further present a body shape data analysis interface, to inform the user that the body shape data is being calculated. For example, the body shape data analysis interface may be a body shape data analysis interface 610 shown in FIG. 6(2). Optionally, the body shape data analysis interface 610 may include a prompt message 611. The prompt message 611 may be used to inform the user that the body shape data is being calculated, and so on.

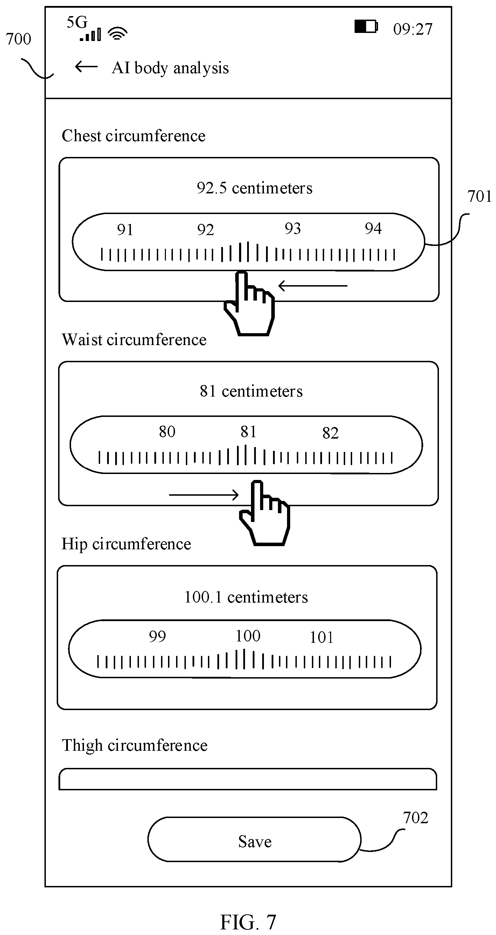

In some embodiments, due to various factors such as a measurement error, a possible difference between a part manually measured by the user and a measurement part defined by the mobile phone (for example, a waist defined by the mobile phone may be a position of the navel, and a waist manually measured by the user may be a position above the navel), different clothing looseness, different angles of view for photographing, and an error in user information, the measured body shape data of the user may be different from actual body shape data of the user, and cannot reflect an actual body shape. Therefore, the user may further modify the measured body shape data, to obtain more accurate body shape data. For example, the user modifies the body shape data on the mobile phone. For example, as shown in FIG. 6(1), the mobile phone detects a tap operation of the user on an edit button 602. In response to the operation, as shown in FIG. 7, the mobile phone displays a body shape data modification interface 700. It may be understood that the operation of enabling the mobile phone to be redirected to the body shape data modification interface 700 described herein is merely an example for description. The operation may alternatively be another operation, for example, a gesture operation, a voice operation, or a button operation. This is not limited in this application.

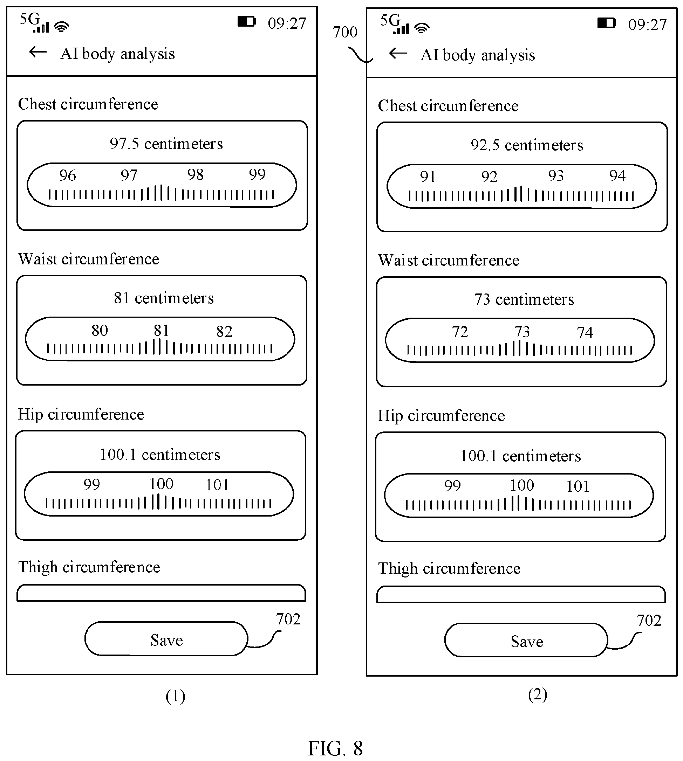

Optionally, on the body shape data modification interface 700, the user may modify, in various manners such as sliding a ruler 701 left or right, one or more pieces of body shape data measured this time. For example, as shown in FIG. 7, the mobile phone detects an operation of sliding a ruler 701 for a chest circumference left by the user. In response to the operation, the mobile phone increases a value of the chest circumference. As shown in (1) in FIG. 8, if the user slides the ruler left to 97.5 centimeters, the mobile phone changes the chest circumference from 92.5 centimeters to 97.5 centimeters. For another example, as shown in FIG. 7, the mobile phone detects an operation of sliding a ruler 701 for a waist circumference right by the user. In response to the operation, the mobile phone decreases a value of the waist circumference. As shown in (2) in FIG. 8, if the user slides the ruler right to 73 centimeters, the mobile phone changes the waist circumference from 81 centimeters to 73 centimeters.

Optionally, the body shape data modification interface 700 may further include a save button 702, and the user may save modified body shape data by using the save button 702.

It may be understood that, the body shape data modification interface 700 shown in FIG. 7 is an example in which a plurality of pieces of body shape data can be simultaneously modified on one interface. Different body shape data may alternatively be modified on a plurality of interfaces. For example, the user may perform a tap operation in a display area of each piece of the body shape data shown in FIG. 6(1). In response to the operation, the mobile phone may display a modification interface corresponding to the body shape data, the user may modify corresponding body shape data (not shown in the figure) on the interface.

In some other embodiments, the mobile phone may further determine the body shape data of the user by combining historical modification data with the solution in the foregoing embodiments. The historical modification data may be a modification amount generated when the user modifies body shape data measured by the mobile phone, for example, a modification amount generated when the user modifies one or more pieces of body shape data in a manner shown in FIG. 7. For example, as shown in (1) in FIG. 8, the user modifies the value of the chest circumference measured by the mobile phone, and a generated modification amount may be +5 centimeters. In other words, historical modification data corresponding to the chest circumference is +5 centimeters. For example, as shown in (2) in FIG. 8, the user modifies the value of the waist circumference measured by the mobile phone, and a generated modification amount is -8 centimeters. In other words, historical modification data corresponding to the waist circumference is -8 centimeters.

It may be understood that historical modification data corresponding to body shape data (for example, the chest circumference, the waist circumference, and the hip circumference) of different parts may be the same or may be different. Optionally, the historical modification data may include historical modification data corresponding to one or more body shape measurement operations.

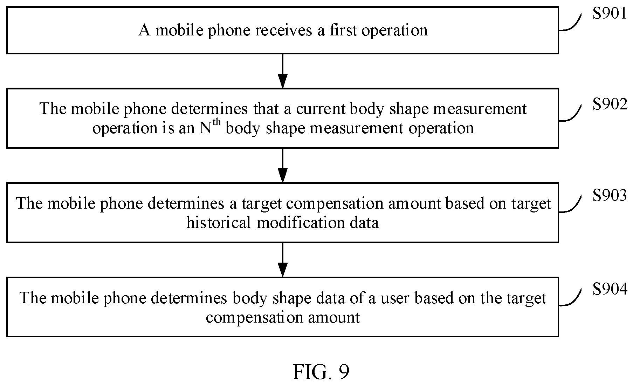

For example, FIG. 9 is a schematic flowchart of a body shape measurement method according to an embodiment of this application. The method includes the following steps.

S901: A mobile phone receives a first operation.

The first operation indicates the mobile phone to perform a body shape measurement operation, or the first operation is used to enable a body shape measurement function of the mobile phone. For example, the first operation may be a tap operation on the photographing start button 301 shown in FIG. 3(1). Certainly, the first operation may alternatively be another gesture operation, a button operation, a voice operation, or the like. A specific type of the first operation is not limited in this application.

S902: The mobile phone determines that a current body shape measurement operation (or referred to as body shape measurement, a body shape measurement process, or the like) is an N^{th} body shape measurement operation.

In some embodiments, N is greater than 1, and N is an integer. In other words, the current body shape measurement operation is not a 1^{st} body shape measurement operation. The current body shape measurement operation is a body shape measurement operation to be performed after the mobile phone receives the first operation.

It may be understood that, in this embodiment, an example in which the current body shape measurement operation is not the 1^{st} body shape measurement operation is used. When the current body shape measurement operation is the 1^{st} body shape measurement operation, because there is no historical modification data, the mobile phone may determine body shape data of the user based on information other than the historical modification data by using the solution described in the foregoing embodiments.

In some other embodiments, if there is historical modification data, for example, there is historical modification data in a cloud or another device, the current body shape measurement operation may alternatively be the 1^{st} body shape measurement operation.

S903: The mobile phone determines a target compensation amount based on target historical modification data.

The target historical modification data is historical modification data corresponding to one or more body shape measurement operations before the N^{th} body shape measurement operation, for example, an (N-1)^{th} body shape measurement operation, an (N-3)^{th} body shape measurement operation, and an (N-4)^{th} body shape measurement operation.

Optionally, the plurality of body shape measurement operations before the N^{th} body shape measurement operation may be successive body shape measurement operations (for example, the (N-3)^{th} body shape measurement operation and the (N-4)^{th} body shape measurement operation), or may not be successive body shape measurement operations (for example, the (N-1)^{th} body shape measurement operation and the (N-3)^{th} body shape measurement operation).

For example, the mobile phone may collect statistics on the target historical modification data, for example, obtain various values such as a sum, a difference, a mode, a median, and an average, and use historical modification data obtained after statistics collection as the target compensation amount. Certainly, the mobile phone may alternatively use specific historical modification data in the target historical modification data as the target compensation amount directly. A specific manner of determining the target compensation amount by the mobile phone is not limited in this application.

In some examples, for body shape data of each part, the mobile phone may determine target historical modification data corresponding to the body shape data, and then determine, based on the target historical modification data corresponding to the body shape data, a target compensation amount corresponding to the body shape data. In other words, the target compensation amount corresponding to the body shape data of each part may be determined based on the target historical modification data corresponding to the part. For example, for a chest circumference, a target compensation amount corresponding to the chest circumference is determined based on target historical modification data of the chest circumference. For a waist circumference, a target compensation amount corresponding to the waist circumference is determined based on target historical modification data of the waist circumference.

In some other examples, for body shape data of a plurality of parts, the mobile phone may alternatively determine target historical modification data corresponding to body shape data of a part, and then determine, based on the target historical modification data, target compensation amounts corresponding to the body shape data of the plurality of parts. In other words, a target compensation amount corresponding to body shape data of a part may be determined based on target historical modification data corresponding to body shape data of another part, or may be determined based on a target compensation amount corresponding to body shape data of another part. For example, for the chest circumference, the target compensation amount corresponding to the chest circumference may be determined based on the target historical modification data of the waist circumference, or may be determined based on a target compensation amount of the waist circumference (for example, the target compensation amount is the same as the target compensation amount of the waist circumference).

In another embodiment, the mobile phone may alternatively determine the target compensation amount in another manner. For example, the mobile phone may determine the target compensation amount based on historical user information (for example, including but not limited to a body weight), historical modification data corresponding to the historical user information, and current user information. It may be understood that the historical user information may be user information corresponding to one or more body shape measurement operations before the N^{th} body shape measurement operation. The current user information may be user information corresponding to the N^{th} body shape measurement operation.

For example, the mobile phone may input the current user information to a pre-trained model, and output the target compensation amount by using the model. Optionally, the model may be a model obtained through training by using the historical user information as an input and the historical modification data corresponding to the historical user information as an output.

S904: The mobile phone determines the body shape data of the user based on the target compensation amount.

In some examples, the mobile phone may compensate target body shape data by using the target compensation amount, to obtain the body shape data of the user. For example, the body shape data of the user may be a sum of the target body shape data and the target compensation amount. Optionally, the target body shape data may be body shape data measured by using the solution described in the foregoing embodiment (for example, body shape data measured based on one or more of a user image and user information that are included but not limited to).

For example, the body shape data of the user includes the chest circumference. If the chest circumference measured in the foregoing embodiment is 110 centimeters, and the target compensation amount corresponding to the chest circumference is 4 centimeters, a chest circumference obtained after compensation is 114 centimeters. Correspondingly, a chest circumference output by the mobile phone, for example, on the body shape data output interface 600 shown in FIG. 6(1), may be 114 centimeters. For another example, the body shape data of the user includes the waist circumference. If the waist circumference measured in the foregoing embodiment is 78 centimeters, and the target compensation amount corresponding to the waist circumference is -8 centimeters, a waist circumference obtained after compensation is 70 centimeters. Correspondingly, a waist circumference output by the mobile phone, for example, on the body shape data output interface 600 shown in FIG. 6(1), may be 70 centimeters.

In some other examples, the mobile phone may alternatively directly input the target compensation amount and the information including but not limited to the one or more of the user image, the user information, and the like that are described in the foregoing embodiment to a pre-trained model, and output the body shape data of the user by using the model.

Based on the foregoing technical solution, with reference to an amount of modification by the user to body shape data historically measured by the mobile phone, a modification amount that may be made by the user to body shape data measured this time by the mobile phone is predicted. The predicted modification amount is used to compensate the body shape data measured this time by the mobile phone, to implement intelligent compensation for the body shape data measured by the mobile phone. In this way, more accurate body shape data can be obtained, and a quantity of modifications of the user can be reduced, thereby bringing convenience to the user and improving user experience.

Optionally, in this embodiment, after determining the body shape data of the user, the mobile phone may also output the body shape data of the user, for example, in a manner shown in FIG. 6(1). Optionally, the user may alternatively modify the output body shape data of the user, for example, in a manner shown in FIG. 7.

It may be understood that, in the solution in this embodiment, the data measured by the mobile phone is corrected based on an amount of manual modification by the user, to improve accuracy of the obtained data. This solution is also applicable to a scenario other than body shape measurement.

In some embodiments, the mobile phone may further determine final current body shape data based on one or more pieces of historical body shape data and the current body shape data. It may be understood that the historical body shape data is body shape data obtained before the current body shape measurement operation. Optionally, the obtained body shape data may be body shape data that is not modified by the user after the mobile phone outputs the determined body shape data, or may be body shape data modified by the user. The current body shape data may be body shape data determined by using the solution described in the foregoing embodiment, for example, includes but is not limited to body shape data determined based on one or more types of information such as the user information, the user image, and the target compensation amount. The final current body shape data may be body shape data that is output by the mobile phone this time and that is not modified by the user.

Based on this solution, the final current body shape data is determined with reference to the historically obtained body shape data and the currently obtained body shape data, so that an accidental error of the body shape data determined this time can be reduced. In this way, when the body shape data is measured a plurality of times in a long term, fluctuation of the body shape data can be smaller, and the body shape data is more consistent with an actual situation.

In some examples, the mobile phone may calculate an average value, another statistical value, or the like of the one or more pieces of historical body shape data and the current body shape data, to determine the final current body shape data.

In some other examples, when determining the final current body shape data based on a plurality of pieces of historical body shape data, the mobile phone may further weight the plurality of pieces of historical body shape data, and then calculate an average value, another statistical value, or the like of weighted historical body shape data and the current body shape data, to determine the final body shape data.

For example, a weight for each piece of historical body shape data may be determined based on a similarity and/or a time difference of user information. The similarity of user information is a similarity between user information corresponding to historical body shape data and user information corresponding to current body shape data. The time difference is a difference between a measurement time point corresponding to historical body shape data and a measurement time point corresponding to the current body shape data. Optionally, a sum of weights for the plurality of pieces of historical body shape data used to determine the final current body shape data may be 1.

For example, a higher similarity of user information indicates a larger weight for corresponding historical body shape data. For example, the user information is a body weight. A body weight corresponding to historical body shape data 1 is 50 kilograms, a body weight corresponding to historical body shape data 2 is 51 kilograms, and a body weight corresponding to current body shape data is 49 kilograms. In this case, a weight for the historical body shape data 1 may be 0.6, and a weight for the historical body shape data 2 may be 0.4.

For another example, when user information has a same similarity, a smaller time difference indicates a larger weight for corresponding historical body shape data. For example, a body weight corresponding to historical body shape data 3, a body weight corresponding to historical body shape data 4, and a body weight corresponding to current body shape data are all 49 kilograms, a measurement date corresponding to the historical body shape data 3 is September 20, a measurement date corresponding to the historical body shape data 4 is September 24, and a measurement date corresponding to the current body shape data is September 27. In this case, a weight for the historical body shape measurement data 3 may be 0.7, and a weight for the historical body shape measurement data 4 may be 0.3.

In some embodiments, the mobile phone may alternatively compensate the current body shape data based on user information corresponding to previous body shape data and user information corresponding to the current body shape data, to determine the final current body shape data. It may be understood that the previous body shape data may be body shape data that is output after a previous body shape measurement operation is performed and that is not modified by the user, or may be body shape data modified by the user. Optionally, a relationship between the final current body shape data and the previous body shape data and a relationship between the user information corresponding to the current body shape data and the user information corresponding to the previous body shape data meet a preset relationship condition.

For example, the user information is a body weight. If a body weight corresponding to the current body shape data is less than a body weight corresponding to the previous body shape data, the final current body shape data is less than or equal to the previous body shape data. Specifically, if the current body shape data is greater than the previous body shape data, the current body shape data is decreased, to obtain the final current body shape data.

For another example, if a body weight corresponding to the current body shape data is greater than a body weight corresponding to the previous body shape data, the final current body shape data is larger or equal to the previous body shape data. Specifically, if the current body shape data is less than the previous body shape data, the current body shape data is increased, to obtain the final current body shape data.

For another example, if a body weight corresponding to the current body shape data is the same as a body weight corresponding to the previous body shape data, a difference between the final current body shape data and the previous body shape data may be in a preset fluctuation range. For example, the preset fluctuation range may be greater than or equal to -1 centimeter and less than or equal to 1 centimeter. In other words, the final current body shape data is at most 1 centimeter greater than the previous body shape data, and at most 1 centimeter less than the previous body shape data.

In other words, a change trend of the body weight is consistent with a change trend of the body shape data as much as possible. For example, both increase or decrease. In this way, consistency between the change trend of the body weight and the change trend of the body shape data can be ensured, so that the obtained body shape data is more accurate and more consistent with an actual situation.

It may be understood that the solution in this embodiment and the solution in the foregoing embodiment may be used separately, or may be used in combination.

In some embodiments, to prevent the obtained body shape data from being inaccurate because the user randomly or incorrectly modifies the body shape data output by the mobile phone, a range available for editing (or referred to as a range available for modification, a range available for edit, or the like) by the user may be further limited. In other words, an amount of a modification by the user to the body shape data falls within the range available for editing. It may be understood that, in this embodiment of this application, the range available for editing may be a range in which the user can modify the body shape data measured by the mobile phone.

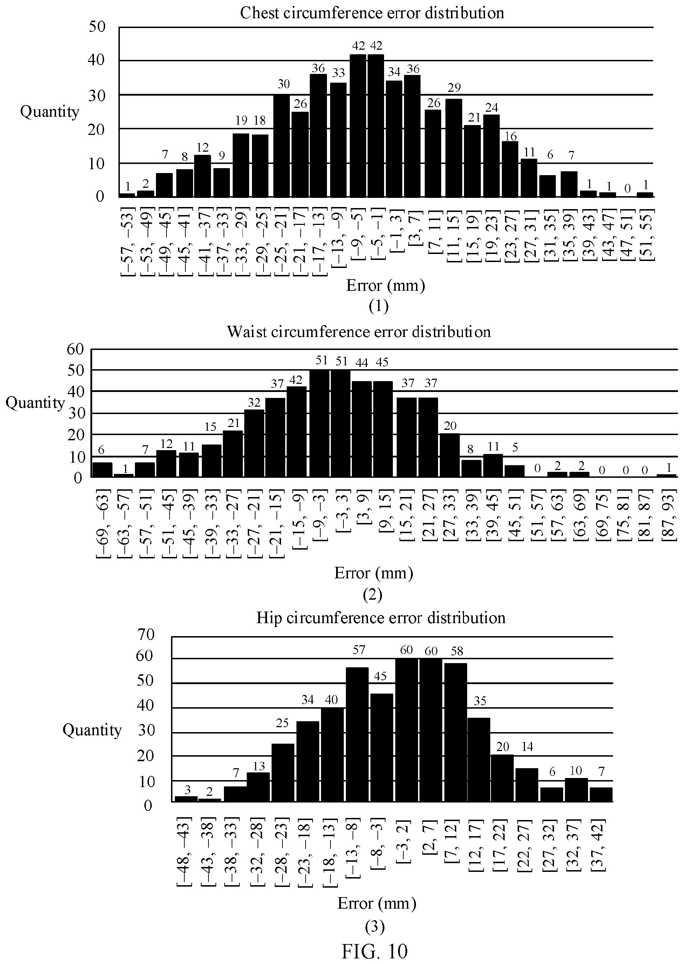

In a specific embodiment, the range available for editing by the user may be determined based on a large quantity of error samples. The error sample may include an error between the body shape data measured by the mobile phone and actual body shape data. For example, FIG. 10 is a diagram of error distribution according to an embodiment of this application. (1) in FIG. 10 is a diagram of distribution of errors between chest circumferences measured by the mobile phone and an actual chest circumference. (2) in FIG. 10 is a diagram of distribution of errors between waist circumferences measured by the mobile phone and an actual waist circumference. (3) in FIG. 10 is a diagram of distribution of errors between hip circumferences measured by the mobile phone and an actual hip circumference. In the diagrams shown in FIG. 10, a horizontal axis represents an error range, and a vertical axis represents a quantity of samples.

In some examples, it can be learned from (1), (2), and (3) in FIG. 10 that the error distribution is normal distribution. Optionally, the range available for editing by the user may be determined based on an expectation and a variance of the normal distribution. For example, the expectation of the normal distribution is u, and the variance is σ2. For example, the range available for editing by the user may be determined based on a corresponding error range. A chest circumference shown in (1) in FIG. 10 is used as an example. For example, the range available for editing by the user may be greater than or equal to -21 millimeters (mm) and less than or equal to 19 mm. In other words, the user may reduce the chest circumference measured by the mobile phone by a maximum of 21 mm, and may increase the chest circumference measured by the mobile phone by a maximum of 19 mm. A waist circumference shown in (2) in FIG. 10 is used as an example. For example, the range available for editing by the user may be greater than or equal to -27 mm and less than or equal to 27 mm. In other words, the user may reduce the waist circumference measured by the mobile phone by a maximum of 27 mm, and may increase the waist circumference measured by the mobile phone by a maximum of 27 mm. A hip circumference shown in (3) in FIG. 10 is used as an example. For example, the range available for editing by the user may be greater than or equal to -18 mm and less than or equal to 22 mm. In other words, the user may reduce the hip circumference measured by the mobile phone by a maximum of 18 mm, and may increase the hip circumference measured by a maximum of 22 mm.

Certainly, the range available for editing by the user may alternatively be determined based on an error range corresponding to (*µ -* 4σ*, µ +* 4*σ*), (*µ -* 2*σ, µ +* 2*σ*), and the like. Alternatively, the range available for editing by the user may be determined based on various statistical values such as an average value, a mode, and a median of the error samples. This is not specifically limited in this application.

Optionally, body shape data of different parts may correspond to a same range available for editing or different ranges available for editing. A range available for editing corresponding to body shape data of a part may be determined based on an error sample corresponding to body shape data of another part, or may be directly determined based on a range available for editing corresponding to body shape data of another part (for example, the range available for editing corresponding to the body shape data of the part is the same as the range available for editing corresponding to the body shape data of the another part), or may be determined based on an error sample corresponding to the body shape data of the part.

In some embodiments, the range available for editing by the user is fixed. In other words, in different body shape measurement processes, ranges available for editing may be the same. For example, in different body shape measurement processes, ranges available for sliding the ruler 701 presented by the mobile phone on the body shape data modification interface 700 shown in FIG. 7 are the same.

In some other embodiments, the range available for editing by the user may dynamically change. In other words, in different body shape measurement processes, ranges available for editing may be different. It may be understood that different ranges available for editing may include the following two cases: Ranges available for editing corresponding to all body shape measurement processes are different from one another, and ranges available for editing corresponding to some body shape measurement processes are the same. For example, in different body shape measurement processes, ranges available for sliding the ruler 701 presented by the mobile phone on the body shape data modification interface 700 shown in FIG. 7 are different.

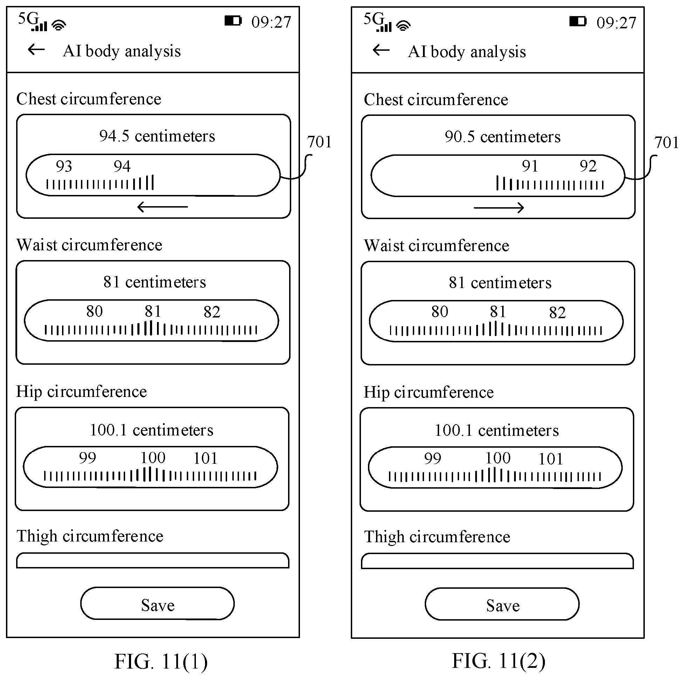

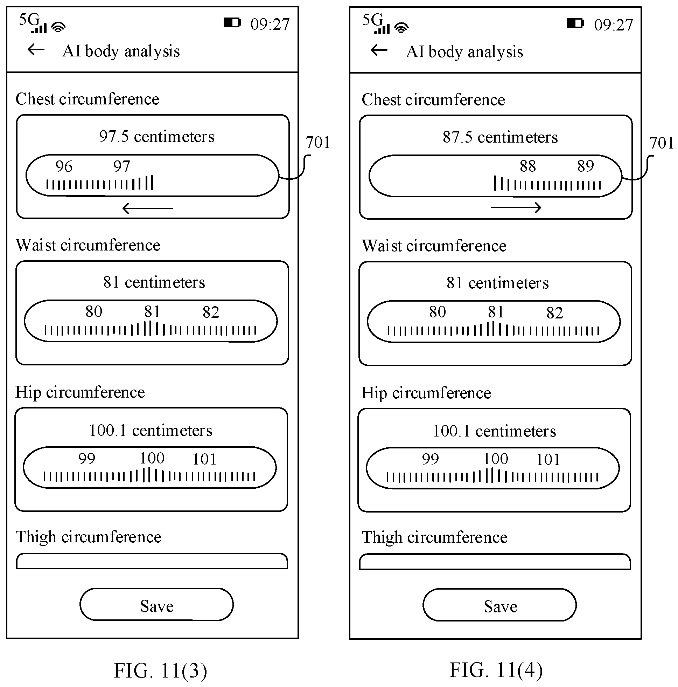

For example, a chest circumference is used as an example. When performing a body shape measurement operation for an a^{th} time, the mobile phone may present the slidable ruler 701 (namely, a first modification range) shown in FIG. 11(1) and FIG. 11(2). As shown in FIG. 11(1), the slidable ruler 701 may be slid left by a maximum of 2 centimeters. In other words, the user may increase a chest circumference measured by the mobile phone by a maximum of 2 centimeters. As shown in FIG. 11(2), the slidable ruler 701 may be slid right by a maximum of 2 centimeters. In other words, the user may reduce the chest circumference measured by the mobile phone by a maximum of 2 centimeters. When performing the body shape measurement operation for a b^{th} time, the mobile phone may present the slidable ruler 701 (namely, a second modification range) shown in FIG. 11(3) and FIG. 11(4). As shown in FIG. 11(3), the slidable ruler 701 may be slid left by a maximum of 5 centimeters. In other words, the user may increase a chest circumference measured by the mobile phone by a maximum of 5 centimeters. As shown in FIG. 11(4), the slidable ruler 701 may be slid right by a maximum of 5 centimeters. In other words, the user may reduce the chest circumference measured by the mobile phone by a maximum of 5 centimeters. Both a and b are positive integers.

Optionally, in this embodiment, an actual length of the slidable ruler 701 may be greater than or equal to the range available for sliding. For example, the length of the slidable ruler 701 may be greater than or equal to 60 centimeters and less than or equal to 100 centimeters, but the range available for sliding the slidable ruler 701 may be only greater than or equal to 80 centimeters and less than or equal to 90 centimeters.

It may be understood that, in this embodiment of this application, different ranges available for editing include but are not limited to one or more of a range available for an increase, a range available for a decrease, and a total range available for an increase and a decrease, and the like. For example, a range 1 available for editing is for an increase of a maximum of 2 centimeters and a decrease of a maximum of 3 centimeters. If a range 2 available for editing is for an increase of a maximum of 2 centimeters and a decrease of a maximum of 4 centimeters, the range 2 available for editing is different from the range 1 available for editing. If a range 3 available for editing is for an increase of a maximum of 5 centimeters and a decrease of a maximum of 3 centimeters, the range 3 available for editing is different from the range 1 available for editing. If a range 4 available for editing is for an increase of a maximum of 3 centimeters and a decrease of a maximum of 5 centimeters, the range 4 available for editing is different from the range 1 available for editing. If a range 5 available for editing is for an increase of a maximum of 3 centimeters and a decrease of a maximum of 2 centimeters, the range 5 available for editing is different from the range 1 available for editing.

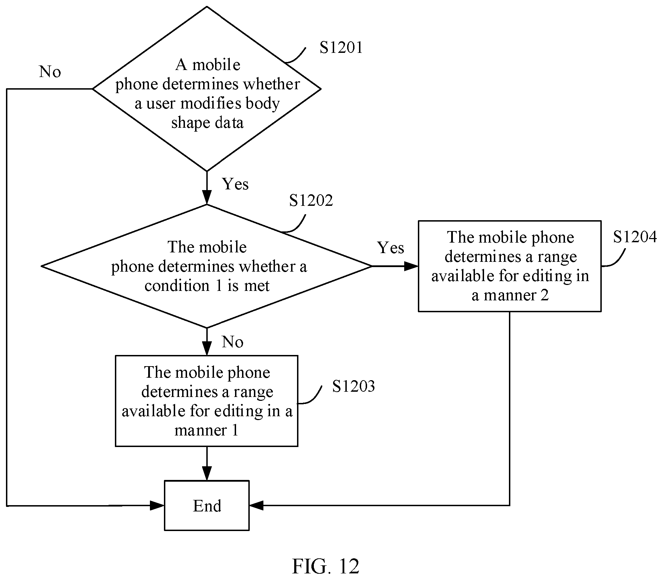

For example, FIG. 12 is a schematic flowchart of a method for determining a range available for editing according to an embodiment of this application. The method includes the following steps.

S1201: A mobile phone determines whether a user modifies body shape data.

If it is determined that the user modifies the body shape data, step S1202 is performed. If it is determined that the user does not modify the body shape data, the process ends.

For example, the user may modify, in a manner shown in FIG. 7, the body shape data output by the mobile phone. Correspondingly, the mobile phone may determine, by detecting whether the user performs a body shape data modification operation, whether the user modifies the body shape data. For example, the body shape modification operation may be a sliding operation on the ruler 701 shown in FIG. 7.

Optionally, the body shape data may be body shape data that is output this time after the mobile phone determines the body shape data by using the solution described in the foregoing embodiment and the like, for example, determines the body shape data based on one or more of user information, a user image, and a target compensation amount that are included but not limited to.

Optionally, when the user does not modify the body shape data, the mobile phone may alternatively determine a range available for editing in a manner 1. For descriptions of the manner 1, refer to the following descriptions.

S1202: The mobile phone determines whether a condition 1 is met.

If the condition 1 is not met, step S1203 is performed. If the condition 1 is met, step S1204 is performed.

In some embodiments, the condition 1 may include one or more of the following: A quantity of times of body shape measurement performed satisfies a preset quantity threshold (for example, greater than or equal to the preset quantity threshold); a difference between user modification amounts corresponding to previous two body shape measurement operations satisfies a preset modification amount difference (or a difference between user modification amounts corresponding to any two successive body shape measurement operations in previous L successive body shape measurement operations satisfies a preset modification amount difference, where L is an integer greater than 2); a similarity between user information (for example, a body weight) corresponding to a current body shape measurement operation and user information corresponding to a previous body shape measurement operation meets a preset similarity condition (for example, less than or equal to a preset similarity threshold); and a user modification amount corresponding to one or more previous body shape measurement operations before a current measurement operation is less than or equal to a preset modification amount, and the like.

For example, the user information is the body weight. If a similarity between a body weight corresponding to the current body shape measurement operation and a body weight corresponding to the previous body shape measurement operation meets the preset similarity condition, it may mean that a difference between the body weight corresponding to the current body shape measurement operation and the body weight corresponding to the previous body shape measurement operation meets the preset similarity condition, for example, is less than or equal to a preset threshold.

Optionally, the mobile phone may compensate the measured body shape data based on a target compensation amount, and the target compensation amount may be determined based on historical modification data of the user. As the amount of manual modification by the user is compensated to the obtained body shape data, the amount of manual modification by the user may become smaller.

S1203: The mobile phone determines the range available for editing in the manner 1.

In some embodiments, the manner 1 is: determining the range available for editing based on an error sample. For a specific implementation, refer to the foregoing descriptions.

It may be understood that, in the solution shown in FIG. 12, the determined range available for editing may be a range available for editing corresponding to the current body shape measurement operation.

S 1204: The mobile phone determines the range available for editing in a manner 2.

In some embodiments, the manner 2 is: determining the range available for editing based on a plurality of types of information in previous body shape data, user information, current body shape data, a predicted error, and the like. For descriptions of the previous body shape data, refer to the foregoing descriptions.

In some implementations, an estimated change amount of the body shape data (or referred to as an estimated body shape change amount) may be determined based on a change amount of the user information (for example, including but not limited to a body weight change amount), and the range available for editing is determined based on the estimated change amount of the body shape data. Alternatively, this is described as determining the range available for editing based on the estimated change amount of the body shape data and the current body shape data. The change amount of the user information may be a change amount of user information corresponding to the current body shape measurement relative to user information corresponding to previous body shape measurement (namely, the previous body shape data).

Optionally, the change amount of the user information and the estimated change amount of the body shape data meet a first preset condition. For example, the first preset condition may include positive correlation. For example, if the change amount of the user information is an increase, the estimated change amount of the body shape data is also an increase. If the change amount of the user information is a decrease, the estimated change amount of the body shape data is also a decrease.

In this implementation, in a specific embodiment, the estimated change amount of the body shape data may be an estimated change amount of the current body shape data relative to the previous body shape data.

In some examples, for example, the user information is a body weight, and the body shape data is a chest circumference. If a body weight corresponding to a chest circumference measured this time is 5 kilograms greater than a body weight corresponding to a previous chest circumference, a chest circumference change amount (namely, an estimated chest circumference change amount) may be 5 centimeters. In other words, the user may increase the chest circumference measured last time by the mobile phone by a maximum of 5 centimeters. If a chest circumference measured last time by the mobile phone is 90 centimeters, and a chest circumference measured this time by the mobile phone is 92 centimeters, the range available for editing may be greater than or equal to 0 centimeters and less than or equal to 3 centimeters. In other words, the user may increase the chest circumference measured this time by a maximum of 3 centimeters. For another example, if a chest circumference measured last time by the mobile phone is 90 centimeters, and a chest circumference measured this time by the mobile phone is 88 centimeters, the range available for editing may be greater than or equal to 0 centimeters and less than or equal to 7 centimeters. In other words, the user may increase the chest circumference measured this time by a maximum of 7 centimeters.

If the body weight corresponding to the chest circumference measured this time is 4 kilograms less than the body weight corresponding to the chest circumference measured last time, the chest circumference change amount may be 4 centimeters. In other words, the user may reduce the chest circumference measured last time by the mobile phone by a maximum of 4 centimeters. If a chest circumference measured last time is 95 centimeters, and a chest circumference measured this time is 98 centimeters, the range available for editing may be greater than or equal to -7 centimeters and less than or equal to 0 centimeters. In other words, the user may reduce the chest circumference measured this time by a maximum of 7 centimeters. For another example, if a chest circumference measured last time is 95 centimeters, and a chest circumference measured this time is 93 centimeters, the range available for editing may be greater than or equal to -2 centimeters and less than or equal to 0 centimeters. In other words, the user may reduce the chest circumference measured this time by a maximum of 2 centimeters.

In another specific embodiment, the estimated change amount of the body shape data may be a change amount available for modifying the body shape data.

In some examples, for example, the user information is a body weight, and the body shape data is a chest circumference. If a body weight corresponding to a chest circumference measured this time is 5 kilograms greater than a body weight corresponding to a previous chest circumference, a chest circumference change amount may be 5 centimeters. In other words, the user may increase the chest circumference measured this time by the mobile phone by a maximum of 5 centimeters. If a chest circumference measured this time is 92 centimeters, the range available for editing may be greater than or equal to 0 centimeters and less than or equal to 5 centimeters. In other words, the user may increase the chest circumference measured this time by a maximum of 5 centimeters.

If the body weight corresponding to the chest circumference measured this time is 4 kilograms less than the body weight corresponding to the chest circumference measured last time, the chest circumference change amount may be 4 centimeters. In other words, the user may reduce the chest circumference measured this time by the mobile phone by a maximum of 4 centimeters. If a chest circumference measured this time is 98 centimeters, the range available for editing may be greater than or equal to -4 centimeters and less than or equal to 0 centimeters. In other words, the user may reduce the chest circumference measured this time by a maximum of 4 centimeters.

It may be understood that, in the foregoing examples, the change amount of the user information is equally proportional to the estimated change amount of the body shape data. Certainly, the change amount of the user information and the estimated change amount of the body shape data may alternatively satisfy another proportional relationship. This is not specifically limited in this application.

In some other implementations, an estimated change amount of the body shape data (or referred to as an estimated body shape change amount) may be determined based on a change amount of the user information (for example, including but not limited to a body weight change amount), and the range available for editing is determined based on a plurality of types of information in the estimated change amount of the body shape data, the predicted error, and the current body shape data. For the change amount of the user information, refer to the descriptions of the foregoing implementation. The estimated change amount of the body shape data may be an estimated change amount of the current body shape data relative to the previous body shape data. The predicted error may be an error between the estimated current body shape data and actual body shape data.

The change amount of the user information and the estimated change amount of the body shape data may also meet a first preset condition. For descriptions of the first preset condition, still refer to the descriptions of the foregoing implementation.

In some examples, an estimated range available for editing may be determined based on the estimated change amount of the body shape data and the predicted error, and then the range available for editing is determined based on the estimated range available for editing and the current body shape data. The estimated range available for editing may be a range in which the estimated current body shape data can be modified.

For example, the user information is still a body weight, and the body shape data is still a chest circumference. If a body weight corresponding to a chest circumference measured this time is 5 kilograms greater than a body weight corresponding to a chest circumference measured last time, a chest circumference change amount (namely, an estimated chest circumference change amount) may be 5 centimeters. If the chest circumference measured last time by the mobile phone is 90 centimeters, the estimated current chest circumference is 95 centimeters. If the predicted error is 3 centimeters, the estimated range available for editing may be greater than or equal to -3 centimeters and less than or equal to 3 centimeters. In other words, the estimated current chest circumference may be increased by a maximum of 3 centimeters, and may be reduced by a maximum of 3 centimeters. In other words, an interval of the estimated range available for editing is [92 centimeters, 95 centimeters].

It may be understood that, in this embodiment of this application, the estimated range available for editing and/or the range available for editing may be represented by an amount available for modifying the body shape data, or may be represented by a sum of the body shape data and an amount available for a modification (for example, in a form of the foregoing interval).

If the current body shape data is in the estimated range available for editing, the range available for editing may be the estimated range available for editing, or may be an intersection of the current body shape data and the estimated range available for editing. If the current body shape is not in the estimated range available for editing, the range available for editing may be a union set of the current body shape data and the range available for editing. For example, if the current chest circumference is 93 centimeters, the range available for editing is [92 centimeters, 95 centimeters], [92 centimeters, 93 centimeters], or [93 centimeters, 95 centimeters]. If the current chest circumference is 100 centimeters, the range available for editing may be [92 centimeters, 100 centimeters], or the like.

For example, the predicted error may be determined in the manner 1, or may be determined based on an empirical value. The predicted error is not limited in this application.

It may be understood that, for body shape data of a same part, if the body shape data measured during different body shape measurement operations is the same, ranges available for editing corresponding to the body shape data may alternatively be different.

Optionally, when the condition 1 is met, the range available for editing may alternatively be determined based on the error sample. This is not limited in this application.

Based on the foregoing technical solution, the range available for editing by the user is limited, to reduce a probability that accuracy of obtained body shape data is reduced because the user randomly or incorrectly edits the obtained body shape data, so that the obtained body shape data is more accurate. In addition, as the amount of manual modification by the user is compensated to the obtained body shape data, the amount of manual modification by the user gradually decreases. Therefore, the range available for editing by the user may be determined based on the plurality of types of information in the previous body shape data, the user information, the current body shape data, the predicted error, and the like. In this way, when the user modifies the body shape data in the range available for editing, consistency between a change trend of the user information and a change trend of the body shape data can be ensured, so that the obtained body shape data is more consistent with a real situation of a body shape change, and precision is higher. In addition, the range available for editing by the user determined based on the previous body shape data and the user information may be smaller. In this way, the user can perform limited editing operations, so that a probability of incorrect editing by the user can be reduced, and editing costs of the user can be reduced.

In another embodiment, the range available for editing may alternatively be determined based on a preset modification range. For example, the preset modification range may be greater than or equal to -3 centimeters and less than or equal to 3 centimeters. In other words, based on the body shape data measured this time, the user may increase the body shape data by a maximum of3 centimeters, and may decrease the body shape data by a maximum of 3 centimeters. Optionally, a specific value of the preset modification range may be set by a developer according to an actual requirement. In some examples, the preset modification range may be set to a small range. In this way, a probability that accuracy of the obtained body shape data is reduced due to random or incorrect editing by the user can be reduced, so that the obtained body shape data is more accurate.

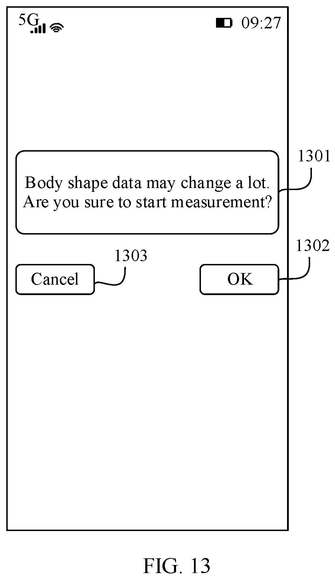

In some embodiments, the mobile phone may further obtain a reference factor, and determine whether the reference factor meets a first condition. When the first condition is met, the body shape data of the user may change. Therefore, the mobile phone may further actively remind the user to perform body shape measurement. For example, as shown in FIG. 13, the mobile phone may display a reminder message 1301, to remind the user to perform body shape measurement. Optionally, the mobile phone may further display an OK button 1302 and/or a cancel button 1303. The mobile phone detects a tap operation of the user on the OK button 1302. In response to the operation, the mobile phone may perform a body shape measurement operation. The mobile phone detects a tap operation of the user on the cancel button 1303. In response to the operation, the mobile phone does not perform a body shape measurement operation.

For example, the reference factor may include but is not limited to one or more of factors such as an amount of exercise, a target body weight change (namely, a second body weight change amount), and a period without a body shape measurement record. For example, the amount of exercise may include an amount of exercise within a period of time, and the amount of exercise may be various kinds of exercise data such as an exercise distance, consumed calories, a quantity of rope jumps, exercise duration, and a quantity of steps. The target body weight change may be a change of an obtained current body weight relative to a body weight obtained last time.

It may be understood that, that the reference factor meets the first condition may mean that each factor included in the reference factor meets a corresponding condition of the factor. For example, a corresponding condition of the amount of exercise may be that a preset condition of the amount of exercise (for example, greater than or equal to a preset amount of exercise threshold) is met. A corresponding condition of the target body weight change may be that a preset body weight condition (for example, greater than or equal to a preset body weight threshold) is met. A corresponding condition of the period without a body shape measurement record may be that a preset period (for example, greater than or equal to the preset period) is satisfied.

Optionally, the mobile phone may monitor the reference factor in real time, periodically, or as scheduled, or may monitor the reference factor when the user starts an application having a body shape measurement function, or may monitor the reference factor when detecting an operation used to modify a body weight included in the user information. An occasion on which the mobile phone monitors the reference factor is not limited in this application.

Optionally, the mobile phone may be associated with at least one electronic device, to obtain the reference factor. For example, the at least one electronic device may include but is not limited to various devices that can be used for measuring and recording exercise data, body weight data, and the like, such as a wearable device (for example, a smart band or a smartwatch), a body fat scale, and a fitness apparatus. Certainly, the mobile phone may further directly obtain the reference factor entered by the user into the mobile phone and/or another device. For example, the reference factor is the body weight. For example, the user may modify the body weight on the profile interface 530 shown in FIG. 5(4). Correspondingly, the mobile phone may obtain a body weight modified by the user, and the modified body weight is the obtained current body weight. The mobile phone may determine the target body weight change based on the modified body weight.

It may be understood that a manner of obtaining the at least one reference factor by the mobile phone is not limited in embodiments of this application.

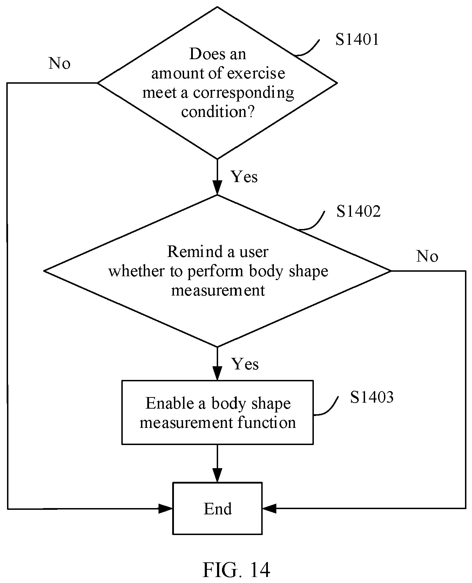

For example, the reference factor is the amount of exercise. FIG. 14 is a schematic flowchart of a method for actively reminding a user to perform body shape measurement according to an embodiment of this application. The method may be performed by a mobile phone, or may be performed by a mobile phone in cooperation with another device. As shown in FIG. 14, the method includes the following steps.

S1401: Determine whether an amount of exercise meets a corresponding condition.

If the condition is met, step S1402 is performed. If the condition is not met, the process ends. For the condition corresponding to the amount of exercise, refer to the foregoing descriptions.

S1402: Remind the user whether to perform body shape measurement.

If the user agrees to perform the body shape measurement, step S1403 is performed. If the user does not agree to perform the body shape measurement, the process ends.

For example, the mobile phone performs this step, and the mobile phone may present a reminder message 1301 shown in FIG. 13, to remind the user whether to perform body shape measurement. For example, if the mobile phone detects a tap operation of the user on the OK button 1302, the mobile phone determines that the user agrees to perform body shape measurement. If the mobile phone detects a tap operation of the user on the cancel button 1303, the mobile phone determines that the user does not agree to perform body shape measurement.

S1403: Enable a body shape measurement function (or referred to as performing a body shape measurement operation).

For a specific implementation of this step, refer to the implementation of determining the body shape data of the user in the foregoing embodiments.

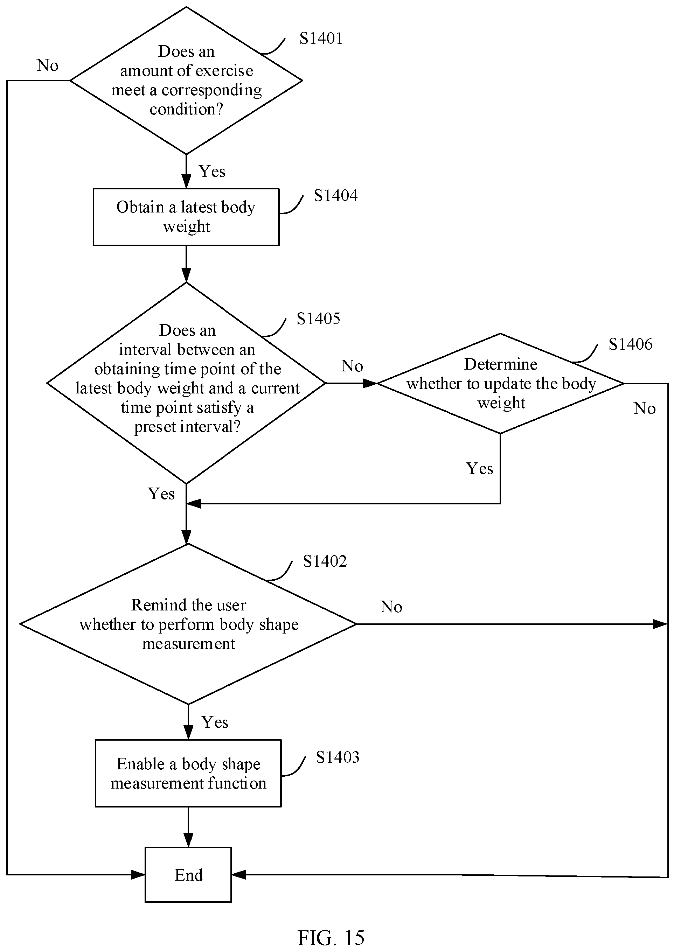

In some embodiments, to improve accuracy of the obtained body shape data, whether user information is recent user information may be further determined. For example, the user information is the body weight. Before step S1402, steps S1404 to S1406 shown in FIG. 15 may be further performed.

S1404: Obtain a latest body weight.

It may be understood that the latest body weight may be a body weight that is obtained at a time point closest to a current time point. For example, the latest body weight may be determined based on a time point (namely, an obtaining time point) corresponding to each body weight. The time point corresponding to each body weight may be determined based on a timestamp of the body weight, or may be determined based on a timestamp of body shape data corresponding to the body weight (namely, a measurement time point of the body shape data).

S1405: Determine whether an interval between the obtaining time point of the latest body weight and the current time point satisfies a preset interval.

If the preset interval is satisfied, step S1402 is performed. If the preset interval is not satisfied, step S1406 is performed.

It may be understood that the current time point may be a time point of current body shape measurement. If the interval between the obtaining time point of the latest body weight and the current time point satisfies the preset interval, it indicates that the latest body weight is a recent body weight. In this way, the body shape data determined based on the recent body weight may be more accurate. If the interval between the obtaining time point of the latest body weight and the current time point does not satisfy the preset interval, it indicates that the latest body weight is not a recent body weight. Consequently, because a difference between a current body weight of the user and the latest body weight of the user may be excessively large, the body shape data determined based on the latest body weight may be inaccurate.

S1406: Determine whether to update the body weight.

This step may be used to obtain the current body weight of the user. For example, the mobile phone performs this step. In some examples, the mobile phone may output a prompt message to prompt the user to enter the current body weight. Correspondingly, the user may determine, based on the prompt message, whether to enter the current body weight. If the user enters the current body weight, after receiving the body weight entered by the user, the mobile phone may perform step S1402. If the user does not enter the current body weight, the process ends. In some other examples, the mobile phone may output a prompt message, to prompt the user to confirm whether to agree to obtain the current body weight. If the user agrees, after obtaining the current body weight of the user, the mobile phone performs step S1402. If the user does not agree, the process ends. For example, the mobile phone may obtain the current body weight of the user from another device. A manner of obtaining the current body weight of the user by the mobile phone is not limited in embodiments of this application.

In some embodiments, step S1406 may be performed. In some other embodiments, step S1406 may not be performed, and the body weight is updated by default. In other words, the current body weight of the user may be directly obtained, and the user does not need to enter the current body weight, or the user agrees to obtain the current body weight.

In some embodiments, after the body weight is updated and before step S1402 is performed, it may be further determined whether a difference between the current body weight and the latest body weight satisfies a preset difference (for example, greater than or equal to the preset difference). When the preset difference is satisfied, step S1402 and subsequent steps are performed. When the preset difference is not satisfied, step S1402 and subsequent steps may not be performed. Optionally, current body shape data of the user may be further determined based on a historical body weight, historical body shape data, and the current body weight. For example, the historical body weight, the historical body shape data, and the current body weight are input to a pre-trained model, and the current body shape data of the user is output by the model. Alternatively, a change amount of the current body shape data relative to the historical body shape data is determined based on a change amount of the current body weight relative to the historical body weight, to determine the current body shape data.

It may be understood that, in the solution shown in FIG. 15, an example in which the user information is the body weight is used. This solution is also applicable to other user information.

Optionally, steps S1404 to S1406 shown in FIG. 15 may alternatively be performed after step S1402 and before step S1403. This is not limited in this application.

It may be understood that, when a reference factor includes one or more of a target body weight change, a period without a body shape measurement record, and the like, for a specific implementation thereof, refer to related implementations of the amount of exercise in FIG. 14 and FIG. 15.

Based on the foregoing technical solution, the user is reminded to actively perform body shape measurement, so that the user can perform a body shape measurement operation based on the reminder, to obtain the body shape data of the user. In this way, the body shape data can be tracked as a long-term indicator, to reflect a body shape change of the user. In addition, compared with that in a case in which the body shape data of the user is not measured for a long time, in a case in which the user is reminded in a timely manner to perform body shape measurement, a body shape measurement frequency can be increased. In this way, precision of the target compensation amount, a range available for editing by the user, and the like can be improved, thereby improving precision of the obtained body shape data.

Optionally, in this embodiment of this application, a determined range available for editing, a determined target compensation amount, and the like may be stored in a database for subsequent use.

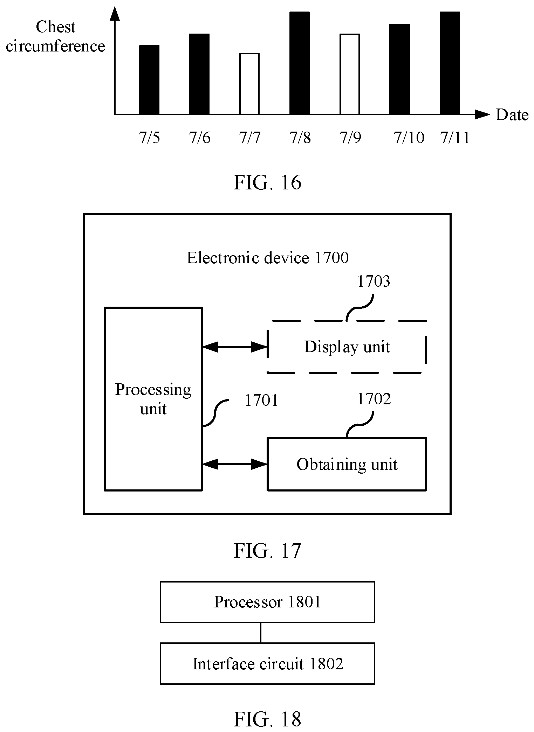

In some embodiments, the mobile phone may further determine, based on the measured body shape data, body shape data in a period in which no body shape measurement operation is performed. For example, a chest circumference is used as an example. As shown in FIG. 16, the chest circumference is not measured on July 7 and July 9, and the chest circumference is measured on July 5, July 6, July 8, July 10, July 11, and the like. Therefore, the mobile phone may estimate chest circumferences on July 7 and July 9 based on chest circumferences measured on one or more of July 5, July 6, July 8, July 10, July 11, and the like.

Optionally, the mobile phone may determine, based on a change amount of target user information (for example, including but not limited to the body weight) and the measured body shape data, the body shape data in the period in which no body shape measurement operation is performed. The change amount of the target user information may be a change amount of user information corresponding to the period in which no body shape measurement operation is performed relative to user information corresponding to the measured body shape data. For example, a change amount of the target user information, the measured body shape data, and the like may be input to a pre-trained model, and the body shape data in the period in which no body shape measurement operation is performed is output by the model. Alternatively, a change amount of the body shape data in the period in which no body shape measurement operation is performed relative to the measured body shape data may be determined based on a change amount of target information, so that the body shape data in the period in which no body shape measurement operation is performed is determined based on the change amount and the measured body shape data.

Based on this solution, the body shape data in the period in which no body shape measurement operation is performed is estimated, so that the user can more clearly view a change of the body shape data. The body shape data in the period in which no body shape measurement operation is performed is determined based on the change amount of the user information, so that the determined body shape data can be consistent with a change trend of the user information, and the body shape data is more authentic.

It may be understood that the solutions in embodiments of this application may be used in any combination in a possible case.

It may be further understood that the interfaces in embodiments of this application are merely diagrams, and do not constitute a limitation on this application. During actual application, more or fewer interfaces may be included, and each interface may alternatively include more or less content.

The foregoing mainly describes the solutions provided in embodiments of this application from the perspective of the methods. It may be understood that, to implement the foregoing functions, the electronic device includes a corresponding hardware structure and/or software module for performing each of the functions. With reference to the units and algorithm steps described in embodiments disclosed in this application, embodiments of this application can be implemented in a form of hardware or combination of hardware and computer software. Whether a function is performed by hardware or hardware driven by a computer depends on particular applications and design constraints of the technical solutions. A person skilled in the art may use different methods to implement the described functions for each particular application, but it should not be considered that the implementation falls beyond the scope of the technical solutions in embodiments of this application.

In embodiments of this application, the electronic device may be divided into function modules based on the foregoing method examples. For example, each function module may be obtained through division based on each corresponding function, or two or more functions may be integrated into one processing unit. The integrated unit may be implemented in a form of hardware, or may be implemented in a form of software functional module. It should be noted that, in embodiments of this application, division into the units is an example, and is merely logical function division. During actual implementation, another division manner may be used.

FIG. 17 is a diagram of a structure of an electronic device according to an embodiment of this application. The electronic device 1700 may be configured to implement the methods recorded in the foregoing method embodiments. For example, the electronic device 1700 may specifically include a processing unit 1701 and an obtaining unit 1702.

The processing unit 1701 is configured to support the electronic device 1700 in performing steps S901 to S904 in FIG. 9; and/or the processing unit 1701 is configured to support the electronic device 1700 in performing steps S1201 to S1204 in FIG. 12; and/or the processing unit 1701 is configured to support the electronic device 1700 in performing steps S1401 to S1403 in FIG. 14; and/or the processing unit 1701 is configured to support the electronic device 1700 in performing steps S1401 to S1403 and S1405 and S1406 in FIG. 15; and/or the processing unit 1701 is further configured to support the electronic device 1700 in performing another step performed by the electronic device in embodiments of this application.

The obtaining unit 1702 is configured to support the electronic device 1700 in performing step S1404 in FIG. 15; and/or the obtaining unit 1702 is configured to support the electronic device 1700 in obtaining a body shape measurement parameter, for example, user information or a user body image; and/or the obtaining unit 1702 is further configured to support the electronic device 1700 in performing another step performed by the electronic device in embodiments of this application.

Optionally, the electronic device 1700 shown in FIG. 17 may further include a display unit 1703. The display unit 1703 may be configured to display one or more of body shape data, a reminder message, and the like; and/or the display unit 1703 is further configured to support the electronic device 1700 in performing other steps performed by the electronic device in embodiments of this application.

Optionally, the electronic device 1700 shown in FIG. 17 may further include a communication unit (not shown in FIG. 17). The communication unit is configured to support the electronic device 1700 in performing a step of communication between the electronic device and another device in embodiments of this application.

Optionally, the electronic device 1700 shown in FIG. 17 may further include a storage unit (not shown in FIG. 17), and the storage unit stores a program or instructions. When the processing unit 1701 executes the program or the instructions, the electronic device 1700 shown in FIG. 17 is enabled to perform the methods shown in FIG. 9, FIG. 12, FIG. 14, FIG. 15, and the like.

For technical effects of the electronic device 1700 shown in FIG. 17, refer to the technical effects of the methods shown in FIG. 9, FIG. 12, FIG. 14, and FIG. 15. Details are not described herein again. The processing unit 1701 in the electronic device 1700 shown in FIG. 17 may be implemented by a processor or a processor-related circuit component, and may be a processor or a processing module. The communication unit may be implemented by a transceiver or a transceiver-related circuit component, and may be a transceiver or a transceiver module. The display unit 1703 may be implemented by a display-related component.

An embodiment of this application further provides a chip system. As shown in FIG. 18, the chip system includes at least one processor 1801 and at least one interface circuit 1802. The processor 1801 and the interface circuit 1802 may be connected to each other through a line. For example, the interface circuit 1802 may be configured to receive a signal from another apparatus. For another example, the interface circuit 1802 may be configured to send a signal to another apparatus (for example, the processor 1801). For example, the interface circuit 1802 may read instructions stored in a memory, and send the instructions to the processor 1801. When the instructions are executed by the processor 1801, an electronic device is enabled to perform the steps performed by the electronic device in the foregoing embodiments. Certainly, the chip system may further include another discrete component. This is not specifically limited in embodiments of this application.

Optionally, there may be one or more processors in the chip system. The processor may be implemented by using hardware, or may be implemented by using software. When the processor is implemented by using hardware, the processor may be a logic circuit, an integrated circuit, or the like. When the processor is implemented by using software, the processor may be a general-purpose processor, and is implemented by reading software code stored in the memory.

Optionally, there may also be one or more memories in the chip system. The memory may be integrated with the processor, or may be disposed separately from the processor. This is not limited in this application. For example, the memory may be a non-transitory processor, for example, a read-only memory ROM. The memory and the processor may be integrated into a same chip, or may be separately disposed on different chips. A type of the memory and a manner of disposing the memory and the processor are not specifically limited in this application.

For example, the chip system may be a field programmable gate array (field programmable gate array, FPGA), an application-specific integrated circuit (application-specific integrated circuit, ASIC), a system on a chip (system on a chip, SoC), a central processing unit (central processing unit, CPU), a network processor (network processor, NP), a digital signal processor (digital signal processor, DSP), a micro controller unit (micro controller unit, MCU), a programmable logic device (programmable logic device, PLD), or another integrated chip.

It should be understood that the steps in the foregoing method embodiments may be completed by using an integrated logic circuit of hardware in the processor or instructions in a form of software. The steps of the method disclosed with reference to embodiments of this application may be directly performed by a hardware processor, or may be performed through a combination of hardware in the processor and a software module.

An embodiment of this application further provides a computer storage medium. The computer storage medium stores computer instructions. When the computer instructions are run on an electronic device, the electronic device is enabled to perform the method in the foregoing method embodiments.

An embodiment of this application provides a computer program product. The computer program product includes a computer program or instructions. When the computer program or the instructions are run on a computer, the computer is enabled to perform the method in the foregoing method embodiments.

In addition, an embodiment of this application further provides an apparatus. The apparatus may be specifically a chip, a component, or a module. The apparatus may include a processor and a memory that are connected to each other. The memory is configured to store computer-executable instructions. When the apparatus runs, the processor may execute the computer-executable instructions stored in the memory, to enable the apparatus to perform the method in the foregoing method embodiments.

The electronic device, the computer storage medium, the computer program product, or the chip provided in embodiments is configured to perform the corresponding method provided above. Therefore, for beneficial effects that can be achieved by the electronic device, the computer storage medium, the computer program product, or the chip, refer to the beneficial effects in the corresponding method provided above. Details are not described herein again.

Based on the descriptions of the foregoing implementations, a person skilled in the art may understand that, for a purpose of convenient and brief description, division into the foregoing functional modules is merely used as an example for illustration. During practical application, the foregoing functions may be allocated to different functional modules and implemented according to a requirement. In other words, an inner structure of an apparatus is divided into different functional modules to implement all or some of the functions described above.

In the several embodiments provided in this application, it should be understood that the disclosed apparatus and method may be implemented in other manners. The embodiments may be combined or referenced with each other without conflict. The described apparatus embodiments are merely examples. For example, the division into modules or units is merely logical function division and may be other division during actual implementation. For example, a plurality of units or components may be combined or integrated into another apparatus, or some features may be ignored or not performed. In addition, the displayed or discussed mutual couplings or direct couplings or communication connections may be implemented by using some interfaces. The indirect couplings or communication connections between the apparatuses or units may be implemented in electronic, mechanical, or other forms.

The units described as separate parts may or may not be physically separate, and parts displayed as units may be one or more physical units, may be located in one place, or may be distributed in a plurality of different places. Some or all of the units may be selected according to actual requirements to achieve the objectives of the solutions of embodiments.

In addition, functional units in embodiments of this application may be integrated into one processing unit, or each of the units may exist alone physically, or two or more units are integrated into one unit. The integrated unit may be implemented in a form of hardware, or may be implemented in a form of software functional unit.

When the integrated unit is implemented in the form of software functional unit and sold or used as an independent product, the integrated unit may be stored in a readable storage medium. Based on such an understanding, the technical solutions of embodiments of this application essentially, or the part contributing to conventional technologies, or all or some of the technical solutions may be implemented in a form of software product. The software product is stored in a storage medium and includes several instructions for instructing a device (which may be a single-chip microcomputer, a chip, or the like) or a processor (processor) to perform all or some of the steps of the method described in embodiments of this application. The storage medium includes various media that can store program code, for example, a USB flash drive, a removable hard disk drive, a read-only memory (read-only memory, ROM), a random-access memory (random-access memory, RAM), a magnetic disk, or an optical disc.

The foregoing descriptions are merely specific implementations of this application, but are not intended to limit the protection scope of this application. Any variation or replacement readily figured out by a person skilled in the art in the technical scope disclosed in this application shall fall within the protection scope of this application. Therefore, the protection scope of this application shall be subject to the protection scope of the claims.

## Claims

1. A body shape measurement method, applied to an electronic device, wherein the method comprises:
obtaining a first body shape measurement parameter, wherein the first body shape measurement parameter comprises user information and a user body image;
determining a first target compensation amount based on first historical modification data, wherein the first historical modification data is an amount of modification by a user to historically measured body shape data;
determining first body shape data based on the first body shape measurement parameter and the first target compensation amount; and
outputting the first body shape data.

2. The method according to claim 1, wherein after the outputting the first body shape data, the method further comprises:
receiving a first user operation, wherein the first user operation is used to modify the first body shape data; and
a range in which the first body shape data is modifiable is a first modification range.

3. The method according to claim 2, wherein after the outputting the first body shape data, the method further comprises:
obtaining a second body shape measurement parameter, wherein the second body shape measurement parameter comprises user information and a user body image;
determining a second target compensation amount based on second historical modification data, wherein the second historical modification data is an amount of modification by the user to historically measured body shape data;
determining second body shape data based on the second body shape measurement parameter and the second target compensation amount; and
outputting the second body shape data.

4. The method according to claim 3, wherein after the outputting the second body shape data, the method further comprises:
receiving a second user operation, wherein the second user operation is used to modify the second body shape data; and
a range in which the second body shape data is modifiable is a second modification range, and the second modification range is different from the first modification range.

5. The method according to any one of claims 2 to 4, wherein the first modification range is determined based on an error sample, and the error sample is an error between body shape data measured by the electronic device and actual body shape data of the user.

6. The method according to claim 4 or 5, wherein the second modification range is determined based on a plurality of types of information in third body shape data, the second body shape data, a first body weight change amount, and a predicted error; the third body shape data is body shape data measured last time before the second body shape data is measured by the electronic device; the first body weight change amount is a change amount of a body weight corresponding to the second body shape data relative to a body weight corresponding to the third body shape data; and the predicted error is an error between estimated second body shape data and the actual body shape data.

7. The method according to claim 6, wherein the second modification range is determined based on a plurality of types of information in an estimated body shape change amount, the predicted error, and the second body shape data; the estimated body shape change amount is a change amount of the estimated second body shape data relative to the third body shape data; and the estimated body shape change amount and the first body weight change amount meet a first preset condition.

8. The method according to claim 4 or 5, wherein the second modification range is determined based on a preset modification range.

9. The method according to any one of claims 1 to 8, wherein the determining first body shape data based on the first body shape measurement parameter and the first target compensation amount comprises:
determining the first body shape data based on the first body shape measurement parameter, the first target compensation amount, and fourth body shape data, wherein the fourth body shape data comprises the historically measured body shape data.

10. The method according to claim 9, wherein the determining the first body shape data based on the first body shape measurement parameter, the first target compensation amount, and fourth body shape data comprises:
determining the first body shape data based on the first body shape measurement parameter, the first target compensation amount, the fourth body shape data, a second body weight change amount, and fifth body shape data, wherein the second body weight change amount is a change amount of a body weight corresponding to the first body shape data relative to a body weight corresponding to the fifth body shape data, and the fifth body shape data is body shape data measured last time before the first body shape data is measured.

11. The method according to any one of claims 1 to 10, wherein the user information comprises one or more of a gender, a height, a body weight, an age, a shoe type, and a clothing type.

12. The method according to any one of claims 1 to 11, wherein the method further comprises:
obtaining a reference factor; and
when the reference factor meets a first condition, reminding the user to perform body shape measurement.

13. The method according to claim 12, wherein the reference factor comprises one or more of the following: an amount of exercise, a third body weight change amount, and a period in which no body shape measurement is performed, wherein the third body weight change amount is a change amount of a current body weight obtained by the electronic device relative to a body weight obtained last time.

14. An electronic device, comprising a processor, a memory, and a display, wherein the memory and the display are coupled to the processor, the memory is configured to store computer program code, the computer program code comprises computer instructions, and when the processor reads the computer instructions from the memory, the electronic device is enabled to perform the following operations:
obtaining a first body shape measurement parameter, wherein the first body shape measurement parameter comprises user information and a user body image;
determining a first target compensation amount based on first historical modification data, wherein the first historical modification data is an amount of modification by a user to historically measured body shape data;
determining first body shape data based on the first body shape measurement parameter and the first target compensation amount; and
outputting the first body shape data.

15. The electronic device according to claim 14, wherein when the processor reads the computer instructions from the memory, the electronic device is further enabled to perform the following operation:
receiving a first user operation, wherein the first user operation is used to modify the first body shape data; and
a range in which the first body shape data is modifiable is a first modification range.

16. The electronic device according to claim 15, wherein when the processor reads the computer instructions from the memory, the electronic device is further enabled to perform the following operations:
obtaining a second body shape measurement parameter, wherein the second body shape measurement parameter comprises user information and a user body image;
determining a second target compensation amount based on second historical modification data, wherein the second historical modification data is an amount of modification by the user to historically measured body shape data;
determining second body shape data based on the second body shape measurement parameter and the second target compensation amount; and
outputting the second body shape data.

17. The electronic device according to claim 16, wherein when the processor reads the computer instructions from the memory, the electronic device is further enabled to perform the following operation:
receiving a second user operation, wherein the second user operation is used to modify the second body shape data; and
a range in which the second body shape data is modifiable is a second modification range, and the second modification range is different from the first modification range.

18. The electronic device according to any one of claims 15 to 17, wherein the first modification range is determined based on an error sample, and the error sample is an error between body shape data measured by the electronic device and actual body shape data of the user.

19. The electronic device according to claim 17 or 18, wherein the second modification range is determined based on a plurality of types of information in third body shape data, the second body shape data, a first body weight change amount, and a predicted error; the third body shape data is body shape data measured last time before the second body shape data is measured by the electronic device; the first body weight change amount is a change amount of a body weight corresponding to the second body shape data relative to a body weight corresponding to the third body shape data; and the predicted error is an error between estimated second body shape data and the actual body shape data.

20. The electronic device according to claim 19, wherein the second modification range is determined based on a plurality of types of information in an estimated body shape change amount, the predicted error, and the second body shape data; the estimated body shape change amount is a change amount of the estimated second body shape data relative to the third body shape data; and the estimated body shape change amount and the first body weight change amount meet a first preset condition.

21. The electronic device according to claim 17 or 18, wherein the second modification range is determined based on a preset modification range.

22. The electronic device according to any one of claims 14 to 21, wherein the determining first body shape data based on the first body shape measurement parameter and the first target compensation amount comprises:
determining the first body shape data based on the first body shape measurement parameter, the first target compensation amount, and fourth body shape data, wherein the fourth body shape data comprises the historically measured body shape data.

23. The electronic device according to claim 22, wherein the determining the first body shape data based on the first body shape measurement parameter, the first target compensation amount, and fourth body shape data comprises:
determining the first body shape data based on the first body shape measurement parameter, the first target compensation amount, the fourth body shape data, a second body weight change amount, and fifth body shape data, wherein the second body weight change amount is a change amount of a body weight corresponding to the first body shape data relative to a body weight corresponding to the fifth body shape data, and the fifth body shape data is body shape data measured last time before the first body shape data is measured.

24. The electronic device according to any one of claims 14 to 23, wherein the user information comprises one or more of a gender, a height, a body weight, an age, a shoe type, and a clothing type.

25. The electronic device according to any one of claims 14 to 24, wherein when the processor reads the computer instructions from the memory, the electronic device is further enabled to perform the following operations:
obtaining a reference factor; and
when the reference factor meets a first condition, reminding the user to perform body shape measurement.

26. The electronic device according to claim 25, wherein the reference factor comprises one or more of the following: an amount of exercise, a third body weight change amount, and a period in which no body shape measurement is performed, wherein the third body weight change amount is a change amount of a current body weight obtained by the electronic device relative to a body weight obtained last time.

27. A computer-readable storage medium, wherein the computer-readable storage medium comprises a computer program or instructions, and when the computer program or the instructions are run on an electronic device, the electronic device is enabled to perform the method according to any one of claims 1 to 13.

28. A computer program product, wherein when the computer program product runs on a computer, the computer is enabled to perform the method according to any one of claims 1 to 13.
